# EUROPEAN PATENT SPECIFICATION

(11) **EP 2 323 661 B1**
(45) Date of publication and mention of the grant of the patent: **17.04.2013**
(21) Application number: 09787530.6
(22) Date of filing: 12.08.2009
(51) Int. Cl.: A61K 31/4706, A61K 31/4709, C07D 401/02, C07D 215/42, C07D 405/12, C07D 215/38, A61P 35/00, A61P 27/06, A61P 37/02

(54) **A3 ADENOSINE RECEPTOR ALLOSTERIC MODULATORS**
ALLOSTERISCHE A3-ADENOSIN-REZEPTOR-MODULATOREN
MODULATEURS ALLOSTÉRIQUES DU RÉCEPTEUR DE L'ADÉNOSINE A3

(30) Priority: 19.08.2008 US 136214 P
(43) Date of publication of application: 25.05.2011
(73) Proprietor: Universiteit Leiden, 2311 RA Leiden (NL)
(72) Inventor: IJZERMAN, Adriaan, NL-2036 MB Haarlem (NL); GOBLYOS, Aniko, H-5052 Ujszasz (HU); BRUSSEE, Johannes, NL-2231 VJ Rijnsburg (NL)
(74) Representative: Jansen, Cornelis Marinus
(86) International application number: PCT/IL2009/000791
(87) International publication number: WO 2010/020981

(56) References cited:
- WO-A1-96/06084
- WO-A1-2007/089507
- US-A1- 2006 194 756
- ARDASHEV B I ET AL: "Isocyanates of the quinoline series" CHEMISTRY OF HETEROCYCLIC COMPOUNDS (A TRANSLATION OF KHIMIYAGETEROTSIKLICHESKIKH SOEDINENII), PLENUM PRESS CO., NEW YORK, NY, US, vol. 8, no. 4, 1 April 1972 (1972-04-01), pages 480-481, XP008114719 ISSN: 0009-3122
- HEITMAN LAURA H ET AL: "A series of 2,4-disubstituted quinolines as a new class of allosteric enhancers of the adenosine A3 receptor." JOURNAL OF MEDICINAL CHEMISTRY 26 FEB 2009, vol. 52, no. 4, 26 February 2009 (2009-02-26), pages 926-931, XP008114805 ISSN: 1520-4804
- "Classical Bioisosteres"; "I" In: A Burger et al.: "Medicinal Chemistry", 1997

## Description

### FIELD OF THE INVENTION

This invention states to A₉ adenosine receptor (A₃AR) allosterio modulators end uses thereof The present invention is defined in the appended claims. Subject matter which is not encompassed by the scope of the claims does not form parts of the present invention.

### PRIOR ART

The following is a list of prior art which is considered to be pertinent for describing the state of the art in the field of the invention. Acknowledgement of these references herein will at times be made by indicating their number within brackets from the list below.
1. Fishman P, et al. Evidence for involvement of Wnt signaling pathway in IB-MECA mediated suppression of melanoma cells. Oncogene., 21:4060-4064 (2002).
2. Fishman P. et al. Targeting the A3 adenosine receptor for cancer therapy: inhibition of Prostate carcinoma cell growth by A3AR agonist. Anticancer Res., 23-2077-2083 (2003).
3. Madi L, et al, A3 adenosine receptor activation in melanoma cells, association between receptor fate and tumor growth inhibition J. Bio. Chem., 278:42121-42130 (2003).
4. Ohana G, et al Inhibition of primary colon carcinoma growth end Hver metastasis by the A3 adenosine receptor agonist IB-MECA. British J. Cancer., 89:1552-1558 (2003).
5. Fishman P. et al. An agonist to the A3 adenosine receptor inhibits colon carcinoma growth in mice via modulation of GSK.-3β and NF-κB. Oncogene, 23-2465-2471 (2004).
6. US Patent Application No. 2004016709 A1.
7. Szabo, C., at al. Suppression of macrophage inflammatory protein (MIP)-1α production and collagen-induced arthritis by adenosine receptor agnnists. British J. Pharmacology, 125:379-397 (1998).
8. Mabley, J., et al. The adenosine A3 receptor agonist, N6-(3-iodobenzyl)-adenosine -5'-N-methyluronamide, is protective in two murine models of colitis. Europ. J. Pharmacology, 466:323-329 (2003).
9. Baharav, E., et al. The effect of adenosine and the A3 adenosine receptor agonist IB-MECA on joint inflammation and autoimmune diseases models. Inter. J. Mol. Med. 10 (supplement 1) page S104, abstract 499 (2002).
10. PCT Application, publication No. WO2005/0063246, entitled "Method for Treatment of Multiple Sclerosis".
11. Montesinos, M. Carmen, et al. Adenosine A2A or A3 receptors are required for inhibition of inflammation by methotrexate and its analog MX-68. Arthritis & Rheumatism, 48:240-247 (2003).
12. Madi L, et al. The A3 Adenosine Receptor is Highly Expressed in Tumor vs. Normal Cells: Potential Target for Tumor Growth Inhibition. Clinical Cancer Research,10: 4472-4479 (2004).
13. US Patent Application, publication No. 20040137477 A1, entitled "A3AR as a marker for a diseased state".
14. Gessi, S. et al. Elevated expression of A3 adenosine receptors in human colorectal cancer is reflected in peripheral blood cells Clinical Cancer Research 10:5895-5901 (2004).
15. Birdsall NJ et al., Allosteric regulation of G-protein-linked receptors Biochem Soc Trans 23:108-111 (1995).
16. Holzgrabe U and Mohr K, Allosteric modulators of ligand binding to muscarinic acetylcholine receptors, Drug Disc Today 3:214-222 (1998).
17. Bruns RF and Fergus JH, Allosteric enhancement of adenosine A1 receptor binding and function by 2-amino-3-benzoylthiophenes, Mol Pharmacol 38:939-949 (1990).
18. Bhattacharya S and Linden J, Effects of long-term treatment with the allosteric enhancer, PD81,723, on Chinese hamster ovary cells expressing recombinant human A1 adenosine receptors, Mol. PharmacoL 50:104-11 (1996).
19. Gao ZG, Kin SG, Soltysiak KA, Melman N, Uzerman AP, Jacobson KA, Selective allosteric enhancement of agonist binding and function at human A3 Adenosine receptors by a series of imidazoquinoline derivatives, Mol Pharmacol 62:81-89 (2002).
20. PCT Application, publication No. WO2007/089507, entitled "A₃ adenosine receptor allosteric modulators".

### BACKGROUND OF THE INVENTION

G protein-coupled receptors (GPCRs) class is the largest family of cell-surface receptors which plays a crucial role in intracellular signal transduction. Adenosine receptors are part of the GPCR class, which belongs to the Class A or rhodopsin-like subfamily of GPCRs. Adenosine, a purine nucleoside, produces numerous physiological actions via cell surface adenosine receptors. These receptors are widely distributed throughout the body and are divided into four subclasses, A₁, A_{2A}, A_{2B} and A₃ receptors, the latter being the most recently identified receptor.

The A₃ adenosine receptor (A₃AR) is involved in a variety of physiological processes. The receptor is highly expressed in various tumor cell types while expression in adjacent normal tissues is relatively low. Activation of the receptor by a specific synthetic agonist induces modulation of downstream signal transduction pathways which include the Wnt and the NF-kB, resulting in tumor growth inhibition (1-5).

*In* vivo studies have shown that A₃AR agonists inhibit the development of colon, prostate and pancreatic carcinomas as well as melanoma and hepatoma. A₃AR agonists were also been shown to act as anti-inflammatory agents by ameliorating the inflammatory process in different experimental autoimmune models such as rheumatoid arthritis, Crohn's disease and multiple sclerosis (6-10). It was proposed also that the A_{2A} and A₃ receptors mediate the anti-inflammatory effects of methotrexate (11).

A₃ adenosine receptor (A₃AR) expression levels are elevated in cancer cells as compared to normal cells (12). Thus, the A₃AR expression level has been described as a means for the diagnosis of cancer (13). In addition, A₃AR expression levels have also been described to be elevated in peripheral blood cells of patients with colorectal cancer (14).

Several members of the GPCR class of receptors have been reported to be modulated allosterically (15), i.e. these receptors have additional binding site(s) on a receptor that are distinct from the agonist binding site (orthosteric site, orthosterically modulated receptors), but that can modulate receptor activity.

Allosteric modulation of GPCRs has been characterized most extensively for muscarinic receptors (16), and it has been suggested that allosteric modulators may provide therapeutic advantages over orthosteric agonists. Such advantages may include greater subtype selectivity and fewer side effects (15).

The adenosine receptors are natural allosteric proteins because agonist-mediated signaling by GPCRs requires a conformational change in the receptor protein transmitted between two topographically distinct binding sites, one for the agonist and another for the G protein. Allosteric sites on GPCRs represent novel drug targets because allosteric modulators possess a number of advantages over classic orthosteric ligands, such as a ceiling level to the allosteric effect and a potential for greater GPCR subtype-selectivity.

Allosteric modulation of A₁ adenosine receptors was reported (17). A number of aminobenzoylthiophenes, including PD81723, were allosteric modulators of the A₁ adenosine receptor. These compounds were shown to be highly subtype-selective enhancers for A₁ adenosine receptors (17) and were less likely to cause desensitization and down-regulation of receptors than selective A₁ adenosine receptor agonists (18).

Some 1H-imidazo-[4,5-c]quinoline derivatives were described as selective allosteric enhancers of human A₃ adenosine receptors (19). Specifically, the derivatives were shown to influence the potency and maximal efficacy of agonist-induced responses while decreasing the dissociation of the agonist N⁶-(4-amino-3-[¹²⁵I]iodobenzyl)-5'-N-methylcarboxamidoadenosine from human A₃ adenosine receptors.

1H-Imidazo-[4,5-c]quinolin-4-amine derivatives were also described for the treatment of conditions which require for said treatment modulations of A₃ adenosine receptor: malignacy, an immuno-compromised affliction, a condition associated with high intraocular pressure (20).

### SUMMARY OF THE INVENTION

The invention provides, in accordance with a first of its aspects, an A₃ adenosine receptor allosteric modulator (A₃RM), having the following general formula (I): 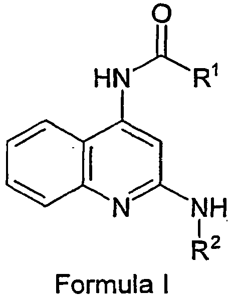 wherein:
- R₁ is a group selected from C₄-C₁₂ cycloalkyl, C₄-C₁₂ cycloalkenyl, C₆-C₁₂ aryl, C₄-C₁₂ heteroaryl, alkcycloalkyl, alkaryl, C₁-C₁₀ alkyl, C₂-C₁₀ alkenyl; C₂-C₁₀ alkynyl, C₅-C₁₅ fused cycloalkyl, bicyclic aromatic or heteroaromatic ring; C₁-C₁₀ alkylether, amino, hydrazido, C₁-C₁₀ alkylamino, C₁-C₁₀ alkoxy, C₁-C₁₀-alkoxycarbony, C₁-C₁₀ alkanol, C₁-C₁₀ acyl, C₁-C₁₀ thioalkoxy, pyridylthio, thio, and C₁-C₁₀ alkylthio, acetoamido and sulfonic acid;
- R₂ is a group selected from hydrogen or a cyclic moiety selected from the group consisting of aryl, heteroaryl, alkaryl, alkheteroaryl, cycloalkyl, heterocycloalkyl, cycloalkenyl, heterocycloalkenyl, cycloalkynyl, heterocycloalkynyl, alkcycloalkyl, alkcycloheteroalkyl, alkcycloalkenyl and alkheterocycloalkenyl said cyclic moiety optionally substituted by at least one group selected from C₁-C₁₀ alkyl, halo, C₁-C₁₀ alkanol, hydroxyl, C₁-C₁₀ acyl, C₁-C₁₀ alkoxy; C₁-C₁₀-alkoxycarbony, C₁-C₁₀ alkoxylalkyl; C₁-C₁₀ thioalkoxy; C₁-C₁₀ alkylether, amino, hydrazido, C₁-C₁₀ alkylamino, pyridylthio, C₂-C₁₀ alkenyl;; C₂-C₁₀ alkynyl, thio, C₁-C₁₀ alkylthio, acetoamido and sulfonic acid; or said substituents can form together with an atom of a cyclic moiety a cycloalkyl, heterocycloalkyl, cycloalkenyl or heterocycloalkenyl fused to said cyclic moiety;
and pharmaceutically acceptable salts thereof, for use in the treatment of a condition which is treatable by a compound selected from the group consisting of adenosine, an A₃ adenosine receptor agonist or an A₃ adenosine receptor antagonist, wherein said condition is selected from immune-compromised afflictions, hyperproliferative disorder, benign hyperplasic disorder, skin proliferative disease, inflammatory disease, ischemic conditions, and conditions associated with high intraocular pressure.

Specific, non-limiting A₃RM according to the invention include a 2,4-disubstituted quinoline derivative selected from:
N-(2-anilinoquinolin-4-yl)cyclopentanecarboxamide
N-{2-[(3,4-dichlorophenyl)amino]quinolin-4-yl}cyclopentanecarboxamide
N-[2-(benzylamino)quinolin-4-yl]cyclopentanecarboxamide
N-{2-[(4-methylphenyl)amino]quinolin-4-yl}cyclopentanecarboxamide
N-[2-(2,3-dihydro-1H-inden-5-ylamino)quinolin-4-yl]cyclopentanecarboxamide
N-{2-[(4-methoxyphenyl)amino]quinolin-4-yl}cyclopentanecarboxamide
N-{2-[(4-chlorophenyl)amino]quinolin-4-yl}cyclopentanecarboxamide
N-[2-(cyclopentylamino)quinolin-4-yl]cyclopentanecarboxamide
N-[2-(1H-indazol-6-ylamino)quinolin-4-yl]cyclopentanecarboxamide
N-(2-anilinoquinolin-4-yl)cyclohexanecarboxamide
N-{2-[(3,4-dichlorophenyl)amino]quinolin-4-yl}cyclohexanecarboxamide
N-{2-[(4-methylphenyl)amino]quinolin-4-yl}cyclohexanecarboxamide
N-[2-(2,3-dihydro-1H-inden-5-ylamino)quinolin-4-yl]cyclohexanecarboxamide
N-(2-anilinoquinolin-4-yl)benzamide
N-{2-[(3,4-dichloro-phenyl)amino]quinolin-4-yl}benzamide
N-(2-anilinoquinolin-4-yl)-2-furamide
N-{2-[(3,4-dichlorophenyl)amino]quinolin-4-yl}-2-furamide
N-(2-anilinoquinolin-4-yl)cyclobutanecarboxamide
N- {2-[(3,4-dichlorophenyl)amino]quinolin-4-yl} cyclobutanecarboxamide.

In one embodiment a 2,4-disubstituted quinoline derivative of the invention is N-{2-[(3,4-dichlorophenyl)amino]quinolin-4-yl}cyclohexanecarboxamide.

The disclosure also concerns an A₃RM for use in enhancing the activity of an A₃ adenosine receptor (A₃AR). In other words, a preferred embodiment of the disclosure concerns an A₃ adenosine receptor (A₃AR) enhancer.

Also the disclosure provides a method of altering/affecting an A₃ adenosine receptor (A₃AR) activity in a subject, the effect being similar to that obtained on said receptor by adenosine or an A₃AR agonist, the method comprises administering to said subject an amount of an A₃ adenosine receptor allosteric modulator (A₃RM), the amount being effective to modulate the A₃AR activity, wherein said A₃RM has the following general formula (I): 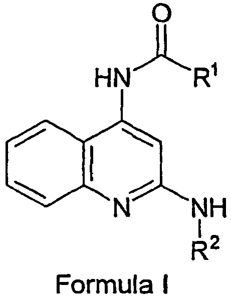 wherein:
- R₁ is a group selected from C₄-C₁₂ cycloalkyl, C₄-C₁₂ cycloalkenyl, C₆-C₁₂ aryl, C₄-C₁₂ heteroaryl, alkcycloalkyl, alkaryl, C₁-C₁₀ alkyl, C₂-C₁₀ alkenyl; C₂-C₁₀ alkynyl, C₅-C₁₅ fused cycloalkyl, bicyclic aromatic or heteroaromatic ring; C₁-C₁₀ alkylether, amino, hydrazido, C₁-C₁₀ alkylamino, C₁-C₁₀ alkoxy, C₁-C₁₀-akoxycarbony, C₁₋C₁₀ alkanol, C₁-C₁₀ acyl, C₁-C₁₀ thioalkoxy, pyridylthio, thio, and C₁-C₁₀ alkylthio, acetoamido and sulfonic acid;
- R₂ is a group selected from hydrogen or a cyclic moiety selected from the group consisting of aryl, heteroaryl alkaryl, alkheteroaryl, cycloalkyl, heterocycloalkyl, cycloalkenyl, heterocycloalkenyl, cycloalkynyl, heterocycloalkynyl, alkcycloalkyl, alkcycloheteroalkyl, alkcycloalkenyl and alkheterocycloakenyl said cyclic moiety optionally substituted by at least one group selected from C₁-C₁₀ alkyl, halo, C₁-C₁₀ alkanol, hydroxyl, C₁-C₁₀ acyl, C₁-C₁₀ alkoxy; C₁-C₁₀-alkoxycarbony, C₁-C₁₀ alkoxylalkyl; C₁-C₁₀ thioalkoxy C₁-C₁₀ alkylether, amino, hydrazido, C₁-C₁₀ alkylamino, pyridylthio, C₂-C₁₀ alkenyl; C₂-C₁₀ alkynyl, thio, C₁-C₁₀ alkylthio, acetoamido and sulfonic acid; or said substituents can form together with an atom of a cyclic moiety a cycloalkyl, heterocycloalkyl, cycloalkenyl or heterocycloalkenyl fused to said cyclic moiety;
and pharmaceutically acceptable salts thereof.

Further provided is a disclosure of method for treating a subject having a condition treatable by adenosine or an A₃AR agonist, the method comprising administering to said subject an amount of an A₃ adenosine receptor allosteric modulator (A₃RM), the amount being sufficient and effective to modulate (change, alter) the A₃AR activity, wherein said A₃RM has the following general formula (I): 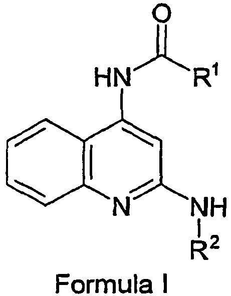
- R₁ is a group selected from C₄-C₁₂ cycloalkyl, C₄-C₁₂ cycloalkenyl, C₆-C₁₂ aryl, C₄-C₁₂ heteroaryl, alkcycloallkyl, alkaryl, C₁-C₁₀ alkyl, C₂-C₁₀ alkenyl; C₂-C₁₀ akynyl, C₅-C₁₅ fused cycloalkyl, bicyclic aromatic or heteroaromatic ring; C₁-C₁₀ alkylether, amino, hydrazido, C₁-C₁₀ alkylamino, C₁-C₁₀ alkoxy, C₁-C₁₀-alkoxycarbony, C₁-C₁₀ alkanol, C₁-C₁₀ acyl, C₁-C₁₀ thioalkoxy, pyridylthio, thio, and C₁-C₁₀ alkylthio, acetoamido and sulfonic acid;
- R₂ is a group selected from hydrogen or a cyclic moiety selected from the group consisting of aryl, heteroaryl alkaryl, alkheteroaryl, cycloalkyl, heterocycloalkyl, cycloalkenyl, heterocycloalkenyl, cycloalkynyl, heterocycloalkynyl, alkcycloalkyl, alkcycloheteroalkyl, alkcycloalkenyl and alkheterocycloalkenyl said cyclic moiety optionally substituted by at least one group selected from C₁-C₁₀ alkyl, halo, C₁-C₁₀ alkanol, hydroxyl, C₁-C₁₀ acyl, C₁-C₁₀ alkoxy; C₁-C₁₀-alkoxycarbony, C₁₋C₁₀ alkoxylalkyl; C₁-C₁₀ thioalkoxy; C₁-C₁₀ alkylether, amino, hydrazido, C₁-C₁₀ alkylamino, pyridylthio, C₂-C₁₀ alkenyl; C₂-C₁₀ alkynyl, thio, C₁-C₁₀ alkylthio, acetoamido and sulfonic acid; or said substituents can form together with an atom of a cyclic moiety a cycloalkyl, heterocycloalkyl, cycloalkenyl or heterocycloalkenyl fused to said cyclic moiety;
and pharmaceutically acceptable salts thereof.

Also provided by the invention is a pharmaceutical composition comprising as active ingredient an A₃RM as defined herein or a 2,4-disubstituted quinoline derivative as provided hereinabove. The pharmaceutical composition is, in accordance with one embodiment, in a form suitable for oral administration.

The invention also provides the use of an A₃RM as defined herein for the preparation of a pharmaceutical composition for the treatment of a condition treatable with adenosine or an A₃AR agonist.

Finally, provided by the invention is a kit comprising an A₃RM as defined herein and instructions for use of said A₃RM in treatment of a condition in a subject which is treatable by adenosine or an A₃AR agonist.

### BRIEF DESCRIPTION OF THE DRAWINGS

In order to understand the invention and to see how it may be carried out in practice, a preferred embodiment will now be described, by way of non-limiting example only, with reference to the accompanying drawings, in which:
**Figure 1** is a scheme of the synthetic procedure of 2,4-disubstituted quinoline derivatives (compounds **16-20).**
**Figure 2** is a scheme of the synthetic procedure of 2,4-disubstituted quinoline derivatives (compounds **21-34).**

### DETAILED DESCRIPTION OF EXEMPLARY EMBODIMENTS

The present invention concerns allosteric modulation (inhibition or enhancement, albeit mostly enhancement) of the A₃ adenosine receptor (A₃AR) by use of 2,4-disubstituted quinoline derivatives. Specifically, the invention is based on the finding that 2,4-disubstituted quinoline derivatives can effectively increase the efficacy of the A₃ adenosine receptor, upon binding thereto.

As appreciated, while the invention is described in the following detailed description with reference to A₃ adenosine receptor modulators for use in treatment, it is to be understood that also encompassed within the present invention are pharmaceutical compositions comprising an A₃ adenosine receptor allosteric modulator, methods making use of such A₃ adenosine receptor allosteric modulators; kits comprising an A₃ adenosine receptor allosteric modulator and instructions for use of the same as well as some novel 2,4-disubstituted quinoline derivatives found to be specifically effective as allosteric modulators, preferably enhancers, of the receptor.

As used herein, the term *"allosteric modulation"* which may be used interchangeably with the term *"allosteric regulation"* denotes the alteration or change (either increase or decrease) in the activity of an enzyme, receptor or other protein by binding of an effector molecule at the A₃ adenosine receptor (A₃AR) allosteric site which is different from the binding site of the endogenous ligand of this A₃AR, the latter being defined as the orthosteric binding site.

Effector molecules that enhance the said activity by binding to the A₃AR allosteric site are referred to herein as *"allosteric activators" or "allosteric enhancers",* whereas those that decrease the activity are called *"allosteric inhibitors".*

Thus, in accordance with a first of its aspects, the present invention provides an A₃ adenosine receptor allosteric modulator (A₃RM) for use in the treatment of a condition which requires for its treatment modulation of an A₃ adenosine receptor (A3AR), and that is treatable with adenosine or an A₃ adenosine receptor (A₃AR) agonist, wherein the A₃RM has the following general formula (I): 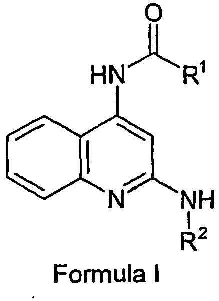 wherein:
- R₁ is a group selected from C₄-C₁₂ cycloalkyl, C₄-C₁₂ cycloalkenyl, C₆-C₁₂ aryl, C₄-C₁₂ heteroaryl, alkcycloalkyl, alkaryl, C₁-C₁₀ alkyl, C₂-C₁₀ alkenyl; C₂-C₁₀ alkynyl, C₅-C₁₅ fused cycloalkyl, bicyclic aromatic or heteroaromatic ring; C₁-C₁₀ alkylether, amino, hydrazido, C₁-C₁₀ alkylamino, C₁-C₁₀ alkoxy, C₁-C₁₀-alkoxycarbony, C₁-C₁₀ alkanol, C₁-C₁₀ acyl, C₁-C₁₀ thioalkoxy, pyridylthio, thio, and C₁-C₁₀ alkylthio, acetoamido and sulfonic acid;
- R₂ is a group selected from hydrogen or a cyclic moiety selected from the group consisting of aryl, heteroaryl, alkaryl, alkheteroaryl, cycloalkyl, heterocycloalkyl, cycloalkenyl, heterocycloalkenyl, cycloalkynyl, heterocycloalkynyl, alkcycloalkyl, alkcycloheteroalkyl, alkcycloalkenyl and alkheterocycloalkenyl said cyclic moiety optionally substituted by at least one group selected from C₁-C₁₀ alkyl, halo, C₁-C₁₀ alkanol, hydroxyl, C₁-C₁₀ acyl, C₁-C₁₀ alkoxy; C₁-C₁₀-alkoxycarbony, C₁-C₁₀ alkoxylalkyl; C₁-C₁₀ thioalkoxy; C₁-C₁₀ alkylether, amino, hydrazido, C₁-C₁₀ alkylamino, pyridylthio, C₂-C₁₀ alkenyl;; C₂-C₁₀ alkynyl, thio, C₁-C₁₀ alkylthio, acetoamido and sulfonic acid; or said substituents can form together with an atom of a cyclic moiety a cycloalkyl, heterocycloalkyl, cycloalkenyl or heterocycloalkenyl fused to said cyclic moiety;
and pharmaceutically acceptable salts thereof, for use in the treatment of a condition which is treatable by a compound selected from the group consisting of adenosine, an A₃ adenosine receptor agonist or an A₃ adenosine receptor antagonist, wherein said condition is selected from immune-compromised afflictions, hyperproliferative disorder, benign hyperplasic disorder, skin proliferative disease, inflammatory disease, ischemic conditions, and conditions associated with high intraocular pressure.

The term *"alkyl"* is used herein to refer to a linear or branched hydrocarbon chain having from 1 to 10 carbon atoms and more preferably 1 to 6 carbon atoms including, but not limited to, methyl, ethyl, n-propyl, isopropyl, n-butyl, t-butyl, n-heptyl, octyl and the like.

Similarly, the therms *"alkenyl"* and *"alkynyl"* denote a linear or branched hydrocarbon chain having, respectively, from 2 to 10, or from 3 to 10 carbon atoms and more preferably 2 to 6 or 3 to 6 carbon atoms, the alkenyl or alkynyl having at least one unsaturated bond.

The alkyl, alkenyl or alkynyl substituents may be substituted with a heteroatom containing group. Thus, it should be understood that while not explicitly stated, any of the alkyl modifications defined hereinabove and below, such as alkylthio, alkoxy, akanol, alkylamine etc, also include the corresponding alkenyl or alkynyl modifications, such as, akenylthio, akenyloxy, alkenol, alkenylamine, or respectively, akynylthio, alkynyloxy, alkynol, alkynylamine.

The term *"aryl"* denotes an unsaturated aromatic carbocyclic group of from 5 to 14 carbon atoms having a single ring (e. g., phenyl) or multiple condensed rings (e. g., naphthyl or anthryl). Preferred aryls include phenyl, indanyl, benzimidazole.

The term *"alkaryl"* refers to -alkylene-aryl groups preferably having from 1 to 10 carbon atoms in the alkylene moiety and from 6 to 14 carbon atoms in the aryl moiety. Such alkaryl groups are exemplified by benzyl, phenethyl and the like.

The term *"substituted aryl"* refers to an aromatic moiety which is substituted with from 1 to 3 substituents as defined above. A variety of substituents are possible, as appreciated by those versed in the art. Nonetheless, some preferred substituents include, without being limited thereto, halogen, (substituted) amino, nitro, cyano, alkyl, alkoxy, acyloxy or alkanol, sulphonyl, sulphynyl.

The term *"halo"* or *"halogen"* refers to fluoro, chloro, bromo and iodo, preferably to chloro.

The term *"acyl"* refers to the groups H-C(O)- as well as alkyl-C(O)-.

The term *"alkanol"* refers to the group -COH as well as alk-OH, "alk" denoting an alkylene, alkenylene or alkynylene chain.

The term *"alkoxy"* is used herein to mean -O-alkyl, including, but not limited to, methoxy, ethoxy, propoxy, isopropoxy, n-butoxy and the like.

The term ***"alkylthio"*** is used herein to mean -S-alkyl, including, but not limited to, methylthio, ethylthio, n-propylthio, isopropylthio, n-butylthio and the like.

The term ***"alkoxyalkyl"*** is used herein to mean -alkyl-O-alkyl, including, but not limited to, methoxymethyl, ethoxymethyl, n-propoxymethyl, isopropoxymethyl, n-butoxymethyl, isobutoxymethyl, t-butoxymethyl and the like.

The term ***"cycloalkyl"*** is used herein to mean cyclic hydrocarbon radicals including, but not limited to, cyclopropyl, cyclobutyl, cyclopentyl, cyclohexyl, cycloheptyl and the like.

The term ***"alkoxycarbonyl"*** is used herein to mean -C(O)O-alkyl, including, but not limited to, methoxycarbonyl, ethoxycarbonyl, propoxycarbonyl and the like.

According to one embodiment of the invention **R¹** is represents a cycloalkyl, aryl or heteroaryl.

In one embodiment **R²** is selected from aryl, alkaryl, cycloalkyl, the aryl or cycloalkyl being optionally substituted by at least one substituent selected from C₁-C₁₀ alkyl, halo (preferably chloro) and C₁-C₁₀ alkylether.

In another embodiment **R¹** is selected from C₄-C₆ cycloalkyl, phenyl or a five membered heterocyclic aromatic ring having the following formula (II): 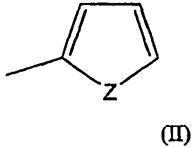
wherein **Z** is selected from O, S or NH; and
**R²** is selected from C₄-C₆ cycloalkyl, phenyl, alkphenyl, or an aromatic ring fused to a five membered cyclic or heteroaromatic ring having the following formulae (IIIa) or (IIIb): 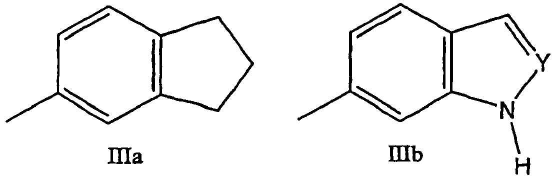 wherein Y is selected from N or CH.
   the aryl or cycloalkyl ring in said cycloalkyl, phenyl, alkphenyl or in formulae (Va) or (Vb) being optionally substituted with a substituent selected from C₁-C₁₀ alkyl, halo, or C₁-C₁₀ alkylether.

In yet another embodiment, **R¹** is selected from C₄-C₆ cycloalkyl, phenyl or a five membered heterocyclic aromatic ring having the following formula (IIa)

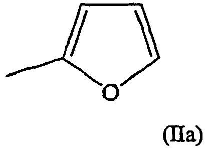

**R²** is selected from cyclopentyl, phenyl, methylphenyl, or an aromatic ring fused to a five membered cyclic or heteroaromatic ring having the following formulae (IIIa) or (IIIb): 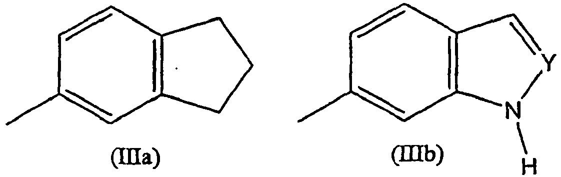 the phenyl being optionally substituted once or more with a methyl, chloro or methylether.

**R²** may also be represented by the general formula (IV): 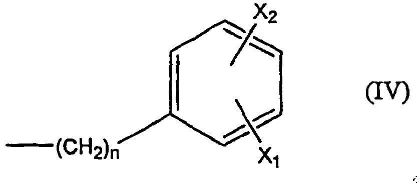 wherein n is 0 or an integer selected from 1-5; preferably, n is 0, 1 or 2; and
- **X₁** and **X₂** which may be the same or different, are selected from hydrogen, halogen, alkyl, alkanol or alkoxy, indanyl, pyrroline provided that when said n is 0, X₁ and X₂ are not hydrogen.

Specific, albeit non-limiting A3RM according to the invention, include the following 2,4-disubstituted quinoline derivatives (in brackets their number according to the following Table 1):
N-(2-anilinoquinolin-4-yl)cyclopentanecarboxamide **(16)**
N-{2-[(3,4-dichlorophenyl)amino]quinolin-4-yl}cyclopentanecarboxamide **(17)**
N-[2-(benzylamino)quinolin-4-yl]cyclopentanecarboxamide **(18)**
N-{2-[(4-methylphenyl)amino]quinolin-4-yl}cyclopentanecarboxamide **(19)**
N-[2-(2,3-dihydro-1H-inden-5-ylamino)quinolin-4-yl]cyclopentanecarboxamide **(20)**
N-{2-[(4-methoxyphenyl)amino]quinolin-4-yl}cyclopentanecarboxamide **(21)**
N-{2-[(4-chlorophenyl)amino]quinolin-4-yl}cyclopentanecarboxamide **(22)**
N-[2-(cyclopentylamino)quinolin-4-yl]cyclopentanecarboxamide **(23)**
N-[2-(1H-indazol-6-ylamino)quinolin-4-yl]cyclopentanecarboxamide **(24)**
N-(2-anilinoquinolin-4-yl)cyclohexanecarboxamide **(25)**
N-{2-[(3,4-dichlorophenyl)amino]quinolin-4-yl}cyclohexanecarboxamide **(26)**
N-{2-[(4-methylphenyl)amino]quinolin-4-yl}cyclohexanecarboxamide **(27).**
N-[2-(2,3-dihydro-1H-inden-5-ylamino)quinolin-4-yl]cyclohexanecarboxamide **(28)**
N-(2-anilinoquinolin-4-yl)benzamide **(29)**
N-{2-[(3,4-dichloro-phenyl)amino]quinolin-4-yl}benzamide **(30)**
N-(2-anilinoquinolin-4-yl)-2-furamide **(31)**
N-{2-[(3,4-dichlorophenyl)amino]quinolin-4-yl}-2-furamide **(32)**
N-(2-anilinoquinolin-4-yl)cyclobutanecarboxamide **(33)**
N-{2-[(3,4-dichlorophenyl)amino]quinolin-4-yl}cyclobutanecarboxamide **(34).**

In one embodiment, said A₃RM is an A₃ adenosine receptor allosteric enhancer, i.e. for use in enhancing the activity of an A₃ adenosine receptor (A₃AR). In accordance with this embodiment, the A₃RM has the above identified formula (I), wherein said **R¹** is a C₄-C₆ cycloalkyl or a phenyl; and **R²** is selected from C₄-C₆ cycloalkyl, phenyl or an aromatic ring fused to a five membered cycloalkyl having the following formulae (III): 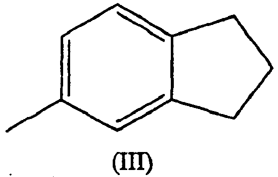 the phenyl moiety in **R²** being unsubstituted or substituted at least once with a C₁-C₃ alkyl, halogen or C₁-C₃ alkether.

In accordance with a more particular embodiment of the A3AR enhancer, the **R¹** is selected from cyclopentyl, cyclohexyl, cyclo butyl or phenyl; and the is selected from cyclopentyl, phenyl or an aromatic ring fused to a five membered cycloalkyl having the following formulae (III): 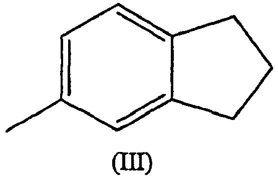 the phenyl moiety in **R²** being unsubstituted or substituted at least once with a methyl, Cl or methylether.

A non-limiting list of A3AR enhancers include the following 2,4-disubstituted quinoline derivatives:
N-{2-[(3,4-dichlorophenyl)amino]quinolin-4-yl}cyclopentanecarboxamide
N-{2-[(4-methoxyphenyl)amino]quinolin-4-yl}cyclopentanecarboxamide
N-{2-[(4-chlorophenyl)amino]quinolin-4-yl}cyclopentanecarboxamide
N-(2-anilinoquinolin-4-yl)cyclohexanecarboxamide
N-{2-[(3,4-dichlorophenyl)amino]quinolin-4-yl}cyclohexanecarboxamide
N-{2-[(4-methylphenyl)amino]quinolin-4-yl}cyclohexanecarboxamide
N-[2-(2,3-dihydro-1H-inden-5-ylamino)quinolin-4-yl]cyclohexanecarboxamide
N-{2-[(3,4-dichlorophenyl)amino]quinolin-4-yl}cyclobutanecarboxamide.

A more specific group of 2,4-disubstituted quinoline derivatives include, without being limited thereto:
N-{2-[(4-chlorophenyl)amino]quinolin-4-yl}cyclopentanecarboxamide
N-(2-anilinoquinolin-4-yl)cyclohexanecarboxamide
N-{2-[(3,4-dichlorophenyl)amino]quinolin-4-yl}cyclohexanecarboxamide
N-[2-(2,3-dihydro-1H-inden-5-ylamino)quinolin-4-yl]cyclohexanecarboxamide.

A preferred A₃AR enhancer is N-{2-[(3,4-dichlorophenyl)amino]quinolin-4-yl}cyclohexanecarboxamide.

With respect to the enhancing activity of the A3RM, the enhancement is also defined by the occurrence of one or more of the following:
- an increase of at least 15% in efficacy of said A₃AR by binding of said A3AR enhancer to an allosteric site of said A₃AR; or
- a decrease in dissociation rate of adenosine or an A₃AR agonist from its binding site;
- less than 60% of displacement of adenosine or an A₃AR agonist from its binding site.

Further, when referring to modulation by enhancement of the activity of the receptor, the condition treatable by adenosine or an A3AR agonist, and to be treated by said allosteric enhancer comprises a malignancy, an immuno-compromised affliction, high intraocular pressure or a condition associated with high intraocular pressure. To this end, the subject requiring said treatment may also be treated in combination with an agonist to the orthosteric binding site of said A₃R.

Conditions for which the A3AR allosteric enhancer is to be used include, rheumatoid arthritis (RA), glaucoma or for enhancing a subject's myeloid system.

The disclosure also concerns a method of affecting an A₃ adenosine receptor (A₃AR) activity in a subject, the effect being similar to that of adenosine or an A₃AR agonist on said receptor, the method comprises administering to said subject an amount of an A₃ adenosine receptor allosteric modulator (A₃RM), the amount being effective to modulate the A₃AR activity, wherein said A₃RM has the general formula (I) as defined above.

When referring to an effect being similar to that of adenosine or an A₃AR agonist on said receptor it is meant that if adenosine and/or an A₃AR agonist increase the activity of an enzyme, protein etc. by binding to the receptor, a similar effect by the A₃RM would be also an increase in the activity of said enzyme, protein etc. The change in activity should be to an extent that a therapeutic effect is achieved by the binding of the A₃RM, the therapeutic effect being defined below with respect to treatment with A₃RM.

Further provided is a disclosure of a method for treating a subject having a condition treatable by adenosine or an A₃AR agonist, the method comprising administering to said subject an amount of an A₃ adenosine receptor allosteric modulator (A₃RM), the amount being effective to modulate the A₃AR activity, wherein said A₃RM has the general formula (I) as defined above.

The term treatment ***"treatment"*** as used herein refers to the therapeutic effect achieved by the administering of an amount of an A₃AM according to the invention and specifically the substituted quinoline derivatives defined herein, the therapeutic effect, being selected from one or more of the following: amelioration of undesired symptoms associated with condition treatable with adenosine or an A₃ adenosine receptor agonist (A₃AR agonist), prevention of the manifestation of such symptoms before they occur, slowing down a progression of the condition, slowing down any deterioration of symptoms of the condition, enhancement of onset of a remission period of a condition, slowing down of any irreversible damage caused in a progressive chronic stage of the condition, delaying of the onset of said progressive stage, lessening of the severity or cure of the condition, improving survival rate or more rapid recovery from the condition, preventing the condition form occurring or a combination of two or more of the above.

A variety of conditions may be treated by the modulation of the A₃AR depending on the specific effect the 2,4-disubstituted quinoline has on the receptor, i.e. inhibition or enhancement.

When modulation comprises ***inhibition*** of or ***decrease*** in efficacy of the receptor, the condition may be any condition treatable by the binding of an A₃ adenosine receptor antagonist. Such conditions comprise certain malignancies or certain immuno-compromised afflictions.

When modulation comprises ***enhancement*** or ***increase*** in efficacy of the receptor, the condition may be any condition which is treatable by the binding of adenosine or an A₃ adenosine receptor agonist. Such conditions comprise hyperproliferative disorders, and in particular all types of solid tumors; skin proliferative diseases (e.g. psoriasis); a variety of benign hyperplasic disorders; inflammatory diseases; ischemic conditions, such as myocardial or renal ischemia and conditions associated with intraocular pressure (e.g. glaucoma).

The term ***"solid tumors"*** refers to carcinomas, sarcomas, adenomas, and cancers of neuronal origin and if fact to any type of cancer which does not originate from the hematopoeitic cells and in particular concerns: carcinoma, sarcoma, adenoma, hepatocellular carcinoma, hepatocellularcarcinoma, hepatoblastoma, rhabdomyosarcoma, esophageal carcinoma, thyroid carcinoma, ganglioblastoma, fibrosarcoma, myxosarcoma, liposarcoma, cohndrosarcoma, osteogenic sarcoma, chordoma, angiosarcoma, endotheliosarcoma, lymphagiosarcoma, synovioama, Ewing's tumor, leimyosarcoma, rhabdotheliosarcoma, colon carcinoma, pancreatic cancer, breast cancer, ovarian cancer, prostate cancer, squamous cell carcinoma, basal cell carcinoma, adenocarcinoma, renal cell carcinoma, hematoma, bile duct carcinoma, melanoma, choriocarcinoma, seminoma, embryonal carcinoma, Wilms' tumor, cervical cancer, testicular tumor, lung carcinoma, small lung carcinoma, bladder carcinoma, epithelial carcinoma, glioma, astrocyoma, medulloblastoma, craniopharyngioma, ependynoma, pinealoma, retinoblastoma, multiple myeloma, rectal carcinoma, thyroid cancer, head and neck cancer, brain cancer, cancer of the peripherial nervous system, cancer of the central nervous system, neuroblastoma, cancer of the endometrium, as well as metastasis of all the above. It has been shown in accordance with the invention that increased expression of A₃AR can be found not only in the primary tumor site but also in metastases thereof.

Benign hyperplasic disorders include benign prostate hyperplasia (BPH), non-tumorigenic polyps in the digestive tract, in the uterus and others.

Inflammatory diseases include rheumatoid arthritis, Crohn's disease, multiple sclerosis and others.

When referring to treatment of a condition treatable by adenosine or an A₃AR agonist, the A₃R enhancer according to the invention is preferably 2,4-disubstituted quinoline derivative selected from:
N-{2-[(3,4-dichlorophenyl)amino]quinolin-4-yl}cyclopentanecarboxamide
N-{2-[(4-methoxyphenyl)amino]quinolin-4-yl}cyclopentanecarboxamide
N-{2-[(4-chlorophenyl)amino]quinolin-4-yl}cyclopentanecarboxamide
N-(2-anilinoquinolin-4-yl)cyclohexanecarboxamide
N-{2-[(3,4-dichlorophenyl)amino]quinolin-4-yl}cyclohexanecarboxamide
N-{2-[(4-methylphenyl)amino]quinolin-4-yl}cyclohexanecarboxamide
N-[2-(2,3-dihydro-1H-inden-5-ylamino)quinolin-4-yl]cyclohexanecarboxamide
N-{2-[(3,4-dichlorophenyl)amino]quinolin-4-yl}cyclobutanecarboxamide.

More preferably, the A₃R enhancer is selected from:
N-{2-[(4-chlorophenyl)amino]quinolin-4-yl} cyclopentanecarboxamide
N-(2-anilinoquinolin-4-yl)cyclohexanecarboxamide
N-{2-[(3,4-dichlorophenyl)amino]quinolin-4-yl}cyclohexanecarboxamide
N-[2-(2,3-dihydro-1H-inden-5-ylamino)quinolin-4-yl]cyclohexanecarboxamide..

According to the invention, the A₃RM may be administered in combination with a ligand to the orthosteric binding site. When modulation involves enhancement of the receptor, the A₃RM may be administered in combination with adenosine or an A₃AR agonist; when modulation involved inhibition of the receptor, the A₃RM may be administered in combination with an A₃AR antagonist

The term ***"combination"*** includes a schedule of treatment that involves administration of at least the A₃RM and the ligand to the orthosteric site. The schedule of treatment may comprise simultaneous or co-administration of the A₃RM and the ligand, or with an interval between administrations. The A₃RM and the ligand may be formulated together or may be included in two different formulations. In addition, the mode of administration and/or the schedule of treatment (i.e. doses per time period) of the A₃RM and the ligand may be different.

According to an aspect of the present invention, the A₃RM is administered to the subject orally; although other administration routes are applicable, including parenteral (intravenous, intramuscular, intra-arterial, subcutaneous, intranasal, via the lungs (inhalation)).

The invention also provides novel 2,4-disubstituted quinoline derivative selected from:
N-(2-anilinoquinolin-4-yl)cyclopentanecarboxamide
N-{2-[(3,4-dichlorophenyl)amino]quinolin-4-yl}cyclopentanecarboxamide
N-[2-(benzylamino)quinolin-4-yl]cyclopentanecarboxamide
N-{2-[(4-methylphenyl)amino]quinolin-4-yl}cyclopentanecarboxamide
N-[2-(2,3-dihydro-1H-inden-5-ylamino)quinolin-4-yl]cyclopentanecarboxamide
N-{2-[(4-methoxyphenyl)amino]quinolin-4-yl}cyclopentanecarboxamide
N-{2-[(4-chlorophenyl)amino]quinolin-4-yl}cyclopentanecarboxamide
N-[2-(cyclopentylamino)quinolin-4-yl]cyclopentanecarboxamide
N-[2-(1H-indazol-6-ylamino)quinolin-4-yl]cyclopentanecarboxamide
N-(2-anilinoquinolin-4-yl)cyclohexanecarboxamide
N-{2-[(3,4-dichlorophenyl)amino]quinolin-4-yl}cyclohexanecarboxamide
N-{2-[(4-methylphenyl)amino]quinolin-4-yl}cyclohexanecarboxamide
N-[2-(2,3-dihydro-1H-inden-5-ylamino)quinolin-4-yl]cyclohexanecarboxamide
N-(2-anilinoquinolin-4-yl)benzamide
N-{2-[(3,4-dichloro-phenyl)amino]quinolin-4-yl}benzamide
N-(2-anilinoquinolin-4-yl)-2-furamide
N-{2-[(3,4-dichlorophenyl)amino]quinolin-4-yl}-2-furamide
N-(2-anilinoquinolin-4-yl)cyclobutanecarboxamide
N-{2-[(3,4-dichlorophenyl)amino]quinolin-4-yl}cyclobutanecarboxamide.

Specifically, the invention provides novel 2,4-disubstituted quinoline derivatives selected from:
N-{2-[(3,4-dichlorophenyl)amino]quinolin-4-yl}cyclopentanecarboxamide
N-{2-[(4-methoxyphenyl)amino]quinolin-4-yl}cyclopentanecarboxamide
N-{2-[(4-chlorophenyl)amino]quinolin-4-yl}cyclopentanecarboxamide
N-(2-anilinoquinolin-4-yl)cyclohexanecarboxamide
N-{2-[(3,4-dichlorophenyl)amino]quinolin-4-yl}cyclohexanecarboxamide
N-{2-[(4-methylphenyl)amino]quinolin-4-yl}cyclohexanecarboxamide
N-[2-(2,3-dihydro-1H-inden-5-ylamino)quinolin-4-yl]cyclohexanecarboxamide
N-{2-[(3,4-dichlorophenyl)amino]quinolin-4-yl}cyclobutanecarboxamide.

More specifically, the invention provides novel 2,4-disubstituted quinoline derivatives selected from:
N-{2-[(4-chlorophenyl)amino]quinolin-4-yl}cyclopentanecarboxamide
N-(2-anilinoquinolin-4-yl)cyclohexanecarboxamide
N-{2-[(3,4-dichlorophenyl)amino]quinolin-4-yl}cyclohexanecarboxamide
N-[2-(2,3-dihydro-1H-inden-5-ylamino)quinolin-4-yl]cyclohexanecarboxamide.

A preferred novel 2,4-disubstituted quinoline derivative according to the invention is N-{2-[(3,4-dichlorophenyl)amino]quinolin-4-yl}cyclohexanecarboxamide.

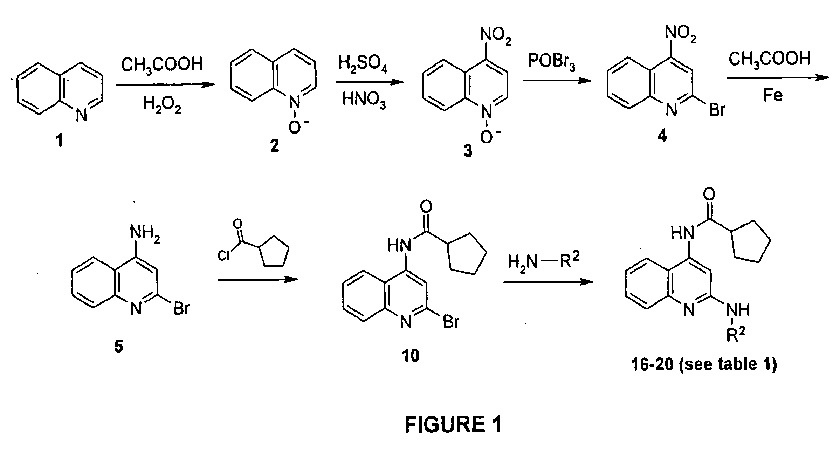

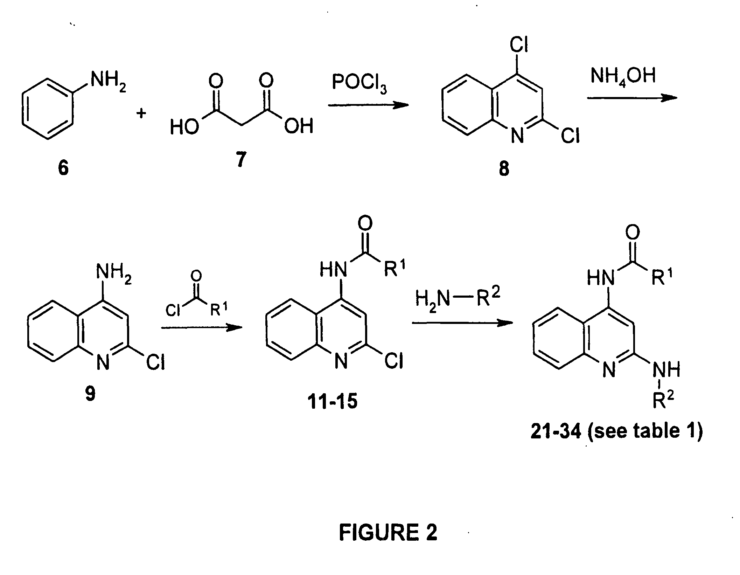

In general, the novel derivatives 16 - 20 were synthesized as shown in the Scheme depicted in **Fig 1****,** while the other novel derivatives **(21-34)** were synthesized as shown in the Scheme depicted in **Fig 2****.** Oxidation of quinoline resulted in quinoline-1-oxide (2) [Ochiai, E. Recent Japanese work on the chemistry of pyridine 1-oxide and related compounds, J. Org. Chem. 1953, 18, 534-551; Zhong, P. et al. A simple and efficient method for the preparation of heterocyclic N-oxide, Synth. Commun. 2004, 34, 247-253], which was nitrated to give 4-nitroquinoline-1-oxide (3) [Taylor Jr, E. C. et al. 3-Methyl-4-nitropyridine-1-oxide. Org. Synth. 1963. Coll. Vol. 4. 654-656; Yokoyama, A. et al. Nitration of quinoline 1-oxide: mechanism of regioselectivity, Chem. Pharm. Bull. 1997, 45, 279-283]. 4-Nitroquinoline-1-oxide (3) was treated with phosphorous oxybromide to afford 2-bromo-4-nitroquinoline (4) [Hamana, M. et al. A new deoxidation reaction of aromatic tertiary amine oxides. Reaction of 4-nitroquinoline 1-oxide with phosphorus bromide. Chem. Abstr. 1957. 51. 6639; Woźniak, M. et al. Amination of 4-nitroquinoline with liquid methylamine/potassium permanganate, Chem. Heterocyc. Comp. 1998, 34, 837-840]. This was converted into 4-amino-2-bromoquinoline (**5**) with iron powder in acetic acid [Kornblum, N. et al. The reduction of optically active 2-nitrooctane and α-phenylnitroethane. J. Am. Chem. Soc. 1955. 77. 6266-6269; Den Hertog, H. J. et al. Reactivity of aminobromoquinolines towards potassium amide in liquid ammonia, Rec. Trav. Chim. des Pays-Bas. 1972, 91, 841-849]. Ring-closure reaction of malonic acid with aniline in phosphorus oxychloride gave 2,4-dichloroquinoline (8) [Osborne, A. G. et al. 2,4-dihalogenoquinolines. Synthesis, orientation effects and 1H and 13C NMR spectral studies, J. Chem. Soc. Perkin Trans. I. 1993, 1, 2747-2755], which was subsequently treated with ammonia in the microwave to give 2-amino-4-chloroquinoline (9) [von Büchi, J. et al. Die tuberkulostatische wirkung von 2-oxy-4-amino-chinolin-derivaten, Helv. Chim. Acta. 1949, 32, 1806-1814; Wojahn, H. Untersuchungen über den zusammenhang von chemischer constitution und anästhesierender wirkung bei 2-alkoxy-chinolin-derivaten, Arch. Pharm. 1936, 274, 83-106]. Reaction of compounds 5 and 9 with carbonyl chlorides [Chang, L. C. W. et al. 2,4,6-Trisubstituted pyrimidines as a new class of selective adenosine A1 receptor antagonists, J. Med. Chem. 2004, 47, 6529-6540] and then subsequently with the appropriate amines afforded the desired compounds **16-20** and **21-34,** respectively [Göblyös, A. et al. Structure-activity relationships of new 1H-imidazo[4,5-c]quinolin-4-amine derivatives as allosteric enhancers of the A3 adenosine receptor, J. Med. Chem. 2006, 49, 3354-3361].

It has now been found that modifying the von Büchi or Wojahn procedure, noted above, by using microwave irradiation (a period of about 2.5 hours max) resulted in an easier and more straightforward purification of the end products. This was unexpected since microwave irradiation has not been considered relevant for this procedure.

It is noted that the above specific 2,4-disubstituted quinoline derivatives are novel *per se* and most have been shown to enhance the response obtained by their allosteric binding to A₃AR.

The 2,4-disubstituted quinoline derivatives of the invention were shown to have, on the one hand, reduced affinity, if any, to the orthosteric binding sites of the A₁, A_{2A}, and A_{2B} adenosine receptors (not shown) and reduced affinity to the orthosteric binding site of the A₃ adenosine receptor (column 4 in Table 2), and on the other hand, high efficacy at the allosteric site of the A₃ adenosine receptor (last column of Table 2). The selective affinity/efficacy of the derivatives disclosed herein is particularly evident with respect to compounds **22, 25, 26,** and **28** in Table 2. These four compounds show little (<50%) displacement of orthosteric ligand binding (column 4 in Table 2), whereas they have obvious enhancing activity (up to 249% compared to a control value of 100% - see last column in Table 2).

As further shown in Table 2 hereinafter, the specific 2,4-disubstituted quinoline derivatives of the invention were shown to increase the activity of the A₃AR. Thus, as indicated above a preferred embodiment of the invention comprises enhancement of A₃AR activity.

Thus, when referring to the substituted quinoline derivatives of formula (I) and the specific novel 2,4-disubstituted quinoline derivatives of the invention, and in line with the above definition of allosteric enhancer, the effect of the substituted quinoline derivatives on the receptor is exhibited by an increase of at least 15% in the efficacy of the A₃ adenosine receptor by binding of the substituted quinoline to the allosteric site of the receptor, which was measured as a decrease (of at least 30%, preferably 40%) in dissociation rate of an A₃AR agonist to the orthosteric binding site.

The invention also provides a pharmaceutical composition comprising as active ingredient a novel 2,4-disubstituted quinoline derivative as provided herein above and below.

Further provided by the invention is a pharmaceutical composition for treating a condition which is treatable with adenosine or an A₃AR agonist, comprising as active ingredient an A₃RM having the formula (I) as defined herein.

The composition of the invention may comprise a combination of A₃RM and a ligand to the orthosteric binding site of said A₃R In one embodiment said ligand is an A₃R agonist and said composition comprises an A₃ adenosine receptor allosteric enhancer.

In one aspect the A₃AM in the composition for treating a condition treatable by adenosine is a 2,4-disubstituted quinoline derivative as disclosed herein.

In one aspect, the pharmaceutical composition of the invention is in a form suitable for oral administration.

The invention further provides a use of the A₃RM having the following general formula (I) and pharmaceutically acceptable salts thereof; for the preparation of a pharmaceutical composition for treatment of a condition which is treatable by adenosine or an A₃AR agonist.

In making the compositions of this invention, the substituted quinoline derivative of formula (I) or the novel 2,4-disubstituted quinoline derivative is usually mixed with the excipient, diluted by an excipient or enclosed within a carrier which can be in the form of a capsule, sachet, paper or other container. The term ***"physiologically acceptable excipient"*** denotes any excipient that is useful in preparing a pharmaceutical composition or formulation that is generally safe, non-toxic and neither biologically nor otherwise undesirable, and includes an excipient that is acceptable for veterinary use as well as human pharmaceutical use. When the excipient serves as a diluent, it can be a solid, semisolid, or liquid material, which acts as a vehicle, carrier or medium for the 2,4-disubstituted quinoline derivative. Thus, the compositions can be in the form of tablets, pills, powders, lozenges, sachets, cachets, elixirs, suspensions, emulsions, solutions, syrups, aerosols (as a solid or in a liquid medium), soft and hard gelatin capsules, suppositories, sterile injectable solutions, and sterile packaged powders.

The effective amount of substituted quinoline derivative of formula (I) or the novel 2,4-disubstituted quinoline derivatives of the invention in the pharmaceutical composition may vary or be adjusted depending upon the particular application, the manner or introduction, the potency of the particular compound, and the desired concentration. The effective amount is typically determined in appropriately designed clinical trials (dose range studies) and the person versed in the art will know how to properly conduct such trials in order to determine the effective amount. As generally known, an effective amount depends on a variety of factors including the affinity of the 2,4-disubstituted quinoline derivative to the allosteric binding site, its distribution profile within the body, a variety of pharmacological parameters such as half life in the body, on undesired side effects, if any, on factors such as age and gender, etc.

The A₃RM is typically administered in unit dosage forms. The term ***"unit dosage forms"*** refers to physically discrete units suitable as unitary dosages for human subjects and other mammals, each unit containing a predetermined quantity of active material calculated to produce the desired therapeutic effect, in association with a suitable pharmaceutical excipient. The amount of therapeutically active compound in such a unit dosage form may vary from about 0.5 mg to 500 mg.

In this case, the composition of the invention will typically be administered over an extended period of time in a single daily dose, in several doses a day, as a single dose and in several days, etc. The treatment period will generally have a length proportional to the length of the disease process and the specific 2,4-disubstituted quinoline derivative effectiveness and the patient species being treated.

In the above and below description and appended claims it is to be understood that the forms ***"a", "an"*** and ***"the"*** include singular as well as plural references unless the context clearly dictates otherwise. For example, the term ***"a 2,4-disubstituted quinoline derivative"*** denotes one or more compounds being the same or different chemical modifications of 2,4-disubstituted quinoline.

Further, it is to be understood that the term ***"comprising"*** is intended to mean that the methods and compositions of the invention may include the recited 2,4-disubstitted quinoline derivative but not excluding other substances. The term ***"consisting essentially of"*** is used to define methods and compositions that include the recited components but exclude other components that may have an essential significance on the biochemical response resulting from the binding of 2,4-disubstituted quinoline derivative to the receptor. For example, a composition consisting essentially of an 2,4-disubstituted quinoline derivative as the active ingredient and a pharmaceutically acceptable carrier will not include or include only insignificant amounts (amounts that will have an insignificant effect on the activity of the receptor) of other compounds capable of binding to the allosteric site or binding site of the receptor. ***"Consisting of"*** shall thus mean excluding more than trace elements of other components. Embodiments defined by each of these transition terms are within the scope of this invention.

Yet further, it is to be understood that all numerical values, e.g. when referring the amounts or ranges of the components constituting the composition of the invention, are approximations which are varied (+) or (-) by up to 20%, at times by up to 10% of from the stated values. It is to be understood, even if not always explicitly stated that all numerical designations are preceded by the term ***"about".***

### SOME EXEMPLARY EMBODIMENTS

### Materials and Methods

### Instruments and Analysis

Microwave-assisted chemistry was performed on an Emrys™ Optimizer with Emrys™ Optimizer software. For the reactions round-bottom vials with a volume of 2-5 mL were used.

¹H-NMR spectra were measured at 200 MHz with a Bruker AC 200 or Bruker DMX 600 spectrometer. ¹³C-NMR spectra were measured at 50 or 150 MHz. Chemical shifts for ¹H and ¹³C are given in ppm (δ) relative to tetramethylsilane (TMS) as internal standard, coupling constants are given in Hz. Melting points were determined with a Büchi capillary melting point apparatus and are uncorrected. Combustion analyses of new target compounds were performed by the analytical department of the Gorlaeus Laboratories, Leiden University (The Netherlands) and are within 0.4 % of theoretical values unless otherwise specified.

### Chemical Synthesis

### Quinoline-1-oxide (2)

Compound 2 was prepared as described elsewhere [Ochiai, E. Recent Japanese Work on the Chemistry of Pyridine 1-oxide and Related Compounds, J. Org. Chem., 1953, 18, 534-551; Zhong, P. et al. A Simple and Efficient Method for the Preparation of Heterocyclic N-oxide. Synth. Commun. 2004, 34, 247-253]. In brief, to a solution of quinoline (25.83 g, 0.2 mol) in acetic acid (70 mL) was added H₂O₂ (35% in water, 1.5 eq, 29 mL) and the reaction stirred at 70 °C for 21 hours. pH was adjusted to 8 with 2M NaOH and the reaction was extracted with DCM (4 x 80 mL). The organic layer was dried over MgSO₄ and evaporated. The product was purified by column chromatography, eluent ethyl acetate. The product was crystallized from ethyl acetate. Yield: 21.72 g (72%). ¹H NMR (CDCl₃) *δ* 7.30 (t, 1H, *J=* 7.66, 6.96 Hz, Ar), 7.61-7.90 (m, 4H, Ar), 8.54 (d, 1H, *J*= 5.84 Hz, Ar), 8.75 (d, 1H, *J*= 8.78 Hz, Ar).

### 4-Nitroquinoline-1-oxide (3)

Compound 3 was prepared by a method described elsewhere [Taylor Jr, E. C. et al. 3-Methyl-4-nitropyridine-1-oxide. Org. Synth. 1963. Coll. Vol. 4. 654-656]. In brief, compound 2 (19.10 g, 0.13 mol) was dissolved in concentrated sulfuric acid and warmed to 65 °C. Nitric acid (65%, 1.1 eq, 15 mL) was added slowly, dropwise. The reaction stirred at 65 °C for 2 hours. The reaction was cooled and poured on ice. The product precipitated as yellow solid, which was filtered off, washed with 5% Na₂CO₃ (1 x 10 mL) water (2 x 10 mL), ethanol (1 x 10 mL) and dried. Yield: 21.90 g (88%). ¹H NMR (CDCl₃) *δ* 7.85-7.94 (m, 2H, Ar), 8.21 (d, 1H, *J=* 6.58 Hz, Ar), 8.53 (d, 1H, *J*= 7.30 Hz, Ar), 8.73-8.86 (m, 1H, Ar). ¹H NMR was identical with ¹H NMR spectrum in literature [Yokoyama, A. et al. Nitration of Quinoline 1-oxide: Mechanism of Regioselectivity. Chem. Pharm. Bull. 1997, 45, 279-283].

### 2-Bromo-4-nitroquinoline (4)

Compound **4** was prepared as described elsewhere [Hamana, M. et al. A new deoxidation reaction of aromatic tertiary amine oxides. Reaction of 4-nitroquinoline 1-oxide with phosphorus bromide. Chem. Abstr. 1957. 51. 6639]. In brief, compound 3 (1.86 g, 9.8 mmol) was dissolved in chloroform and cooled in an ice-bath. POBr₃ (1.25 eq) was added and the reaction stirred in an ice-bath for 2 hours. The reaction was poured on ice, pH was adjusted to 9 with 2M NaOH and extracted with DCM (3 x 80 mL). The product was purified by column chromatography, eluent DCM. Yield: 1.30 g (52 %). ¹H NMR (CDCl₃) *δ* 7.63-7.94 (m, 2H, Ar), 8.06 (s, 1H, Ar), 8.17 (t, 1H, *J=* 7.16, 6.84Hz, Ar), 8.38 (d, 1H, *J*= 8.76 Hz, Ar). ¹H NMR was identical with ¹H NMR spectrum in literature [Woźniak, M. et al. Amination of 4-nitroquinoline with Liquid Methylamine/Potassium permanganate. Chem. Heterocyc. Comp. 1998, 34, 837-840].

### 4-Amino-2-bromoquinoline (5)

Compound 5 was prepared by a method described elsewhere [Kornblum, N. et al. The reduction of optically active 2-nitrooctane and α-phenylnitroethane. J. Am. Chem. Soc. 1955. 77. 6266-6269; Den Hertog, H. J.; Buurman, D. J. Rec. Trav. Chim. des Pays-Bas. 1972, 91, 841-849]. In brief, compound 4 (1.82 g, 7.2 mmol) was dissolved in acetic acid. Iron powder (5 eq) was added and the reaction stirred at 65 °C for 2.5 hours. The iron powder was filtered off, washed with DCM. pH was adjusted to 9 with 2M NaOH. This was filtered again and the residue was washed with ammonia. The aqueous layer was extracted with DCM, dried on MgSO₄ and evaporated. The product was purified by column chromatography, eluent DCM. Yield: 0.69 g (43%). ¹H NMR (CDCl₃) *δ* 4.81 (bs, 2H, NH₂), 6.76 (s, 1H, Ar), 7.48 (t, 1H, *J* = 7.31, 7.06 Hz, Ar), 7.62-7.73 (m, 2H, Ar), 7.94 (d, 1H, *J=* 8.76 Hz, Ar). ¹³C NMR (CDCl₃) *δ* 106.60, 117.82, 120.21, 125.37, 129.19, 130.40, 142.59, 148.78, 150.90.

### 2,4-Dichloroquinoline (8)

Compound 8 was prepared as described elsewhere [Osborne, A. G. et al. 2,4-Dihalogenoquinolines. Synthesis, Orientation Effects and 1H and 13C NMR Spectral Studies. J. Chem. Soc. Perkin Trans. I. 1993, 1, 2747-2755], In brief, malonic acid (8.32 g, 0.08 mol) was dissolved in POCl₃ (60 mL) and cooled in an ice-bath. Aniline (1.25 eq) was added dropwise. The reaction was refluxed for 2.5 hours, then it was cooled to room temperature and poured on ice. pH was adjusted to 9 with 2M NaOH. The precipitate was filtered off and the aqueous layer was extracted with DCM. The product was purified by column chromatography, eluent DCM. Yield: 5:51 g (35%). ¹H NMR (CDCl₃) *δ* 1.25 (s, 1H, Ar), 7.64 (t, 1H, *J*= 7.84, 6.16 Hz, Ar), 7.79 (t, 1H, *J=* 6.89, 6.71 Hz, Ar), 8.03 (d, 1H, *J* = 8.16 Hz, Ar), 8.21 (d, 1H, *J* = 8.21 Hz, Ar). ¹H NMR spectrum was identical with ¹H NMR spectrum in literature.

### 4-Amino-2-chloroquinoline (9)

Preparation of compound **9** is described elsewhere [von Büchi, J. et al. Die Tuberkulostatische Wirkung von 2-Oxy-4-amino-chinolin.Derivaten. Helv. Chim. Acta. 1949, 32, 1806-1814; Wojahn, H. Untersuchungen über den Zusammenhang von chemischer Konstitution und anästhesierender Wirkung bei 2-Alkoxy-chinolin Deivaten. Arch. Pharm. 1936, 274, 83-106]. However, we prepared compound **9** as described here. In brief, compound **8** (0.35 g, 1.8 mmol) was suspended in ammonia (28-30% in water, 3 mL). The reaction was carried out in the microwave at 160 °C for 2.5 hours. After the reaction was completed, ammonia was evaporated off. The product was purified by column chromatography, eluent 3% MeOH in DCM. Yield: 0.14 g (44%). ¹H NMR (CDCl₃) *δ* 4.83 (bs, 2H, NH₂), 6.62 (s, 1H, Ar), 7.43-7.51 (m, 1H, Ar), 7.63-7.73 (m, 2H, Ar), 7.89-7.95 (m, 1H, Ar). ¹³C NMR (600MHz, CDCl₃) *δ* 103.07, 117.60, 120.08, 125.27, 129.13, 130.46, 148.26, 151.40, 151.41.

### N-(2-bromoquinolin-4-yl)cyclopentanecarboxamide (10)

In brief, compound 5 (0.32 g, 1.40 mmol) was dissolved in pyridine (5 mL) and cyclopenthanecarbonyl chloride (1.3 eq) was added. The reaction stirred at 115 °C for 2 hours. After the reaction was completed, pyridine was evaporated. The product was purified by column chromatography, eluent 5% MeOH in DCM. The product was crystallized from MeOH to give white crystals. Yield: 0.35 g (79%). MS (ESI) m/z: 319.9 [M+H]⁺¹, [M-H]⁺¹. ¹H NMR (CDCl₃) *δ* 1.64-2.07 (m, 8H, 4CH₂), 2.53-3.00 (m, 1H, CH), 7.54-7.62 (m, 1H, Ar), 7.69-7.79 (m, 2H, Ar), 7.92 (bs, 1H, NH), 7.99-8.08 (m, 1H, Ar), 8.43 (s, 1H, Ar). ¹³C NMR (CDCl₃) *δ* 25.96, 30.51, 47.2, 114.51, 118.85, 126.76, 129.89, 130.40, 141.56, 143.17, 148.63, 175.07 [Chang, L. C. W. et al. 2,4,6-Trisubstituted pyrimidines as a new class of selective adenosine A1 receptor antagonists, J. Med. Chem. 2004, 47, 6529-6540].

### General procedure for the preparation of N-(2-chloroquinolin-4-yl)carboxamides (11-15)

In brief, compound 9 was dissolved in pyridine (1 mmol/3 mL) and the appropriate acid chloride (1.3 eq) was added. The reaction was stirred at 60 °C for 90 minutes. After the reaction was completed, pyridine was evaporated. The product was purified by column chromatography [Chang, L. C. W. et al. 2,4,6-Trisubstituted pyrimidines as a new class of selective adenosine A1 receptor antagonists, J. Med. Chem. 2004, 47, 6529-6540].

### N-(2-chloroquinolin-4-yl)cyclopentanecarboxamide (11)

Scale: 1.6 mmol. Eluent for column chromatography was 5% MeOH in DCM. Yield: 0.34 g (78%). ¹H NMR (CDCl₃) *δ* 1.58-2.18 (m, 8H, 4CH₂), 2.81-2.98 (m, 1H, CH), 7.53-7.61 (m, 1H, Ar), 7.68-7.76 (m, 2H, Ar), 7.95-8.03 (m, 2H, Ar, NH), 8.42 (s, 1H, Ar). ¹³C NMR (CDCl₃) *δ* 26.18, 30.72, 47.43, 91.47, 94.32, 111.33, 118.94, 126.86, 130.04, 130.65, 142.41, 148.26, 152.24, 175.28.

### N-(2-chloroquinolin-4-yl)cyclohexanecarboxamide (12)

Scale: 2.2 mmol. Eluent for column chromatography was 2-5% MeOH in DCM. The product was crystallized from MeOH to give white crystals. Yield: 0.60 g (95%). ¹H NMR (CDCl₃) *δ* 1.28-2.04 (m, 10H, 5CH₂), 2.38-2.53 (m, 1H, CH), 7.56-7.63 (m, 1H, Ar), 7.71-7.79 (m, 2H, Ar), 7.96-7.63 (m, 2H, Ar, NH), 8.44 (s, 1H, Ar). ¹³C NMR (CDCl₃) *δ* 25.51, 29.66, 46.83, 111.21, 118.52, 118.73, 126.67, 129.89, 130.46, 142.17, 148.05, 152.09, 174.71.

### N-(2-chloro-quinolin-4-yl)benzamide (13)

Scale: 3.91 mmol. Eluent for column chromatography was DCM. Yield: 0.47 g (42%). ¹H NMR (CDCl₃) *δ* 7.52-7.65 (m, 4H, Ar), 7.72-7.84 (m, 2H, Ar), 7.94-8.06 (m, 3H, Ar), 8.51 (s, 1H, Ar), 8.64 (bs, 1H, NH). ¹³C NMR (CDCl₃) *δ* 111.40, 118.83, 126.50, 126.89, 128.83, 129.38, 130.26, 132.56, 133.47, 141.93, 147.72, 151.48, 165.67.

### N-(2-chloroquinolin-4-yl)-2-furamide (14)

Scale: 3.36 mmol. Eluent for column chromatography was DCM. Yield: 0.52 g (57%). ¹H NMR (CDCl₃) *δ* 6.65-6.68 (m, 1H, Ar), 7.39 (d, 1H, *J*= 3.65 Hz, Ar), 7.60-7.90 (m, 4H, Ar), 8.05 (d, 1H, *J*= 12.00 Hz, Ar), 8.52 (s, 1H, Ar), 8.93 (bs, 1H, NH). ¹³C NMR (CDCl₃) *δ* 110.49, 112.98, 116.77, 118.10, 118.59, 126.60, 129.42, 130.33, 141.36, 144.94, 146.58, 147.67, 151.49, 155.65.

### N-(2-chloroquinolin-4-yl)cyclobutanecarboxamide (15)

Scale: 3.92 mmol. Eluent for column chromatography was 1% MeOH in DCM. Yield: 0.89 g (87%). ¹H NMR (CDCl₃ + 1 drop of MeOD) *δ* 1.72-2.58 (m, 6H, 3CH₂), 3.28-3.45 (m, 1H, CH), 7.52-7.60 (m, 1H, Ar), 7.69-7.83 (m, 3H, Ar, NH), 7.98-8.03 (m, 1H, Ar), 8.43 (s, 1H, Ar). ¹³C NMR (CDCl₃+1 drop of MeOD) *δ* 17.85, 25.04, 40.74, 111.13, 118.38, 118.89, 126.35, 129.29, 130.23, 142.09, 147.73, 151.58, 173.81.

### General procedure for the preparation of 2,4-substituted quinolines (16-34)

*Method A:* Compounds **10-15** were dissolved/suspended in absolute ethanol (1.5 mmol/2.5 mL) and the appropriate amines (3 eq) were added. The mixture was heated in the microwave at 140 °C for 80 min. After the reaction was completed, ethanol was evaporated and the residue was dissolved in DCM (100 mL) and washed with 1 M NaOH (3 x 100 mL). The organic layer was dried on MgSO₄. The products were purified by column chromatography and recrystallized [Göblyös, A. et al. Structure-activity relationships of new 1H-imidazo[4,5-c]quinolin-4-amine derivatives as allosteric enhancers of the A3 adenosine receptor, J. Med Chem. 2006, 49, 3354-3361].

*Method B:* Compounds **10-15** and the appropriate amines (10 eq) were heated in the microwave without any solvent at 180 °C for 90 min. After the reaction was completed, the reaction mixture was dissolved in DCM (100 mL) and washed with water (2 x 50 mL), brine (1 x 50 mL). The organic layer was dried on MgSO₄. The products were purified by column chromatography and recrystallized.

### N-(2-anilinoquinolin-4-yl)cyclopentanecarboxamide (16)

*Method A.* Scale: 0.36 mmol of compound **10**. Eluent for column chromatography was 3-10% MeOH in DCM. The product was recrystallized from methanol to give yellow crystals. Yield: 0.039 g (33%). MS (ESI) m/z: 331.2 [M+H]⁺¹. ¹H NMR (CDCl₃) *δ* 1.72-2.04 (m, 8H, 4CH2), 2.80-2.98 (m, 1H, CH), 6.93 (br s, 1H, NH), 7.07 (t, 1H, J = 5.74, 8.06 Hz, Ar), 7.25-7.41 (m, 3H, Ar), 7.57-7.67 (m, 5H, m; Ar), 7.78-7.83 (m, 1H, Ar), 8.01 (s, 1H, NH). ¹³C NMR (CDCl₃) *δ* 25.63, 30.18, 46.89, 100.90, 116.18, 118.36, 119.73, 122.50, 127.25, 129.46, 139.77, 140.96, 147.54, 154.51, 174.87. Anal. calcd for C₂₁H₂₁N₃O·0.3H₂O C, 74.89; H, 6.46; N, 12.48. Found C, 74.89; H, 6.81; N, 12.18.

### N-{2-[(3,4-dichlorophenyl)amino]quinolin-4-yl}cyclopentanecarboxamide (17)

*Method B.* Scale: 0.34 mmol of compound 10. Eluent for column chromatography was 2% MeOH in DCM. The product was recrystallized from methanol to give white crystals. Yield: 0.024 g (19%). MS (ESI) m/z: 400.3 [M+H]⁺¹. ¹H NMR (CDCl₃) *δ* 1.61-2.01(m, 8H, 4CH₂), 2.80-2.97 (m, 1H, CH), 6.79 (bs, 1H, NH), 7.32-7.51 (m, 3H, Ar), 7.57-7.67 (m, 2H, Ar), 7.81-7.94 (m, 3H, Ar, NH), 8.08 (s, 1H, Ar). ¹³C NMR *δ* (CDCl₃+1 drop of MeOD) *δ* 25.84, 30.45, 46.40, 102.78, 117.03, 118.30, 119.49, 120.40, 122.97, 124.13, 127.22, 129.68, 130.04, 132.10, 140.41, 141.32,147.35, 154.21,176.62. Anal. calcd for C₂₁H₁₉N₃OCl_{2˙}0.55CH₂Cl₂C, 59.38; H, 4.53; N, 9.40. Found C, 59.00; H, 4.43; N, 9.72.

### N-[2-(benzylamino)quinolin-4-yl]cyclopentanecarboxamide (18)

*Method A.* Scale: 0.35 mmol of compound **10** and 3 eq of benzylamine_{˙}HCl. Eluent for column chromatography was 3-5% MeOH in DCM. The product was recrystallized from methanol to give yellow crystals. Yield: 0.040 g (33%). MS (ESI) m/z: 345.4 [M+H]⁺¹. ¹H NMR (CDCl₃) *δ* 1.65-2.09 (m, 8H, 4CH₂), 2.79-2.92 (m, 1H, CH), 4.73 (d, 2H, *J*= 8.0 Hz, CH₂), 5.06 (bs, 1H, NH), 7.20-7.41 (m, 7H, Ar), 7.51-7.59 (m, 2H, Ar); 7.70 (s, 1H, Ar), 7.74 (s, 1H, NH). ¹³C NMR (CDCl₃) *δ* 25.66, 30.24, 45.40, 46.92, 100.11, 115.82, 118.27, 121.52, 126.92, 127.10, 127.49, 128.28, 129.25, 139.11, 140.44, 148.42, 157.18, 174.98. Anal. calcd for C₂₂H₂₃N₃O·0.4H₂O C, 74.93; H, 6.80; N, 11.92. Found C, 74.86; H, 6.76; N, 12.16.

### N-{2-[(4-methylphenyl)amino]quinolin-4-yl}cyclopentanecarboxamide (19)

*Method A.* Scale: 0.36 mmol of compound **10.** Eluent for column chromatography was 0-2% MeOH in DCM. The product was recrystallized from methanol to give light brown crystals. Yield: 0.050 g (40%). MS (ESI) m/z: 345.4 [M+H]⁺¹. ¹H NMR (CDCl₃) *δ* 1.56-2.05 (m, 8H, 4CH₂), 2.31 (s, 3H, CH₃), 2.72-2.88 (m, 1H, CH), 6.87 (bs, 1H, NH), 7.10-7.28 (m, 3H, Ar), 7.43-7.57 (m, 4H, Ar), 7.73-7.89 (m, 3H, Ar, NH). ¹³C NMR (CDCl₃) *δ* 20.26, 25.42, 29.97, 46.61, 100.53, 116.03, 118.21, 120.03, 122.00, 127.16, 129.10, 131.98, 137.01, 140.56, 147.81, 154.78, 174.77. Anal. calcd. for C₂₂H₂₃N₃O·0.5H₂O C, 74.55; H, 6.82; N, 11.86. Found C, 74.38; H, 6.81; N, 11.94.

### N-[2-(2,3-dihydro-1H-inden-5-ylamino)quinolin-4-yl]cyclopentanecarboxamide (20)

*Method A.* Scale: 0.35 mmol of compound **10.** Eluent for column chromatography was 5% MeOH in DCM. The product was recrystallized from methanol to give brown crystals. Yield: 0.054 g (45%). MS (ESI) m/z: 371.5 [M+H]⁺¹. ¹H NMR (CDCl₃) *δ* 1.59-2.13 (m, 8H, 4CH₂), 2.74-2.90 (m, 6H, 3CH₂), 6.93 (bs, 1H, NH), 7.14-7.31 (m, 4H, Ar), 7.42-7.58 (m, 3H, Ar), 7.75 (d, 1H, *J*= 8.58 Hz, Ar), 7.88 (s, 1H, NH). ¹³C NMR (CDCl₃) *δ* 25.60, 25.90, 30.45, 32.27, 33.03, 47.07, 101.02, 116.42, 117.00, 118.64, 118.85, 122.43, 124.58, 127.58, 129.58, 138.05, 139.05, 140.93, 145.23, 148.29, 155.42, 175.22. Anal. calcd. for C₂₄H₂₅N₃O·1.5H₂O C, 72.34; H, 7.08; N, 10.54. Found C, 72.47; H, 6.84; N, 10.32.

### N-{2-[(4-methoxyphenyl)amino]quinolin-4-yl}cyclopentanecarboxamide (21)

*Method A.* Scale: 0.84 mmol of compound **11.** Eluent for column chromatography was 3% MeOH in DCM. The product was recrystallized from methanol to give brown crystals. Yield: 0.12 g (39%). MS (ESI) m/z: 361.4 [M+H]⁺¹. ¹H NMR (CDCl₃) *δ* 1.60-2.06 (m, 8H, 4CH₂), 2.77-2.95 (m, 1H, CH), 3.82 (s, 3H, CH₃), 6.69 (bs, 1H, NH), 6.88-6.97 (m, 2H, Ar), 7.23-7.31 (m, 1H, Ar), 7.44-7.60 (m, 4H, Ar), 7.73-7.90 (m, 3H, Ar, NH). ¹³C NMR (CDCl₃) *δ* 25.93, 30.48, 47.25, 55.50, 100.32, 114.48, 116.30, 118.42, 122.43, 122.91, 127.70, 129.68, 133.01, 140.96, 148.45, 155.79, 155.97, 175.10. Anal. calcd. for C₂₂H₂₃N₃O₂·0.5H₂O C, 71.53; H, 6.53; N, 11.34. Found C, 71.55; H, 6.44; N, 11.36.

### N-{2-[(4-chlorophenyl)amino]quinolin-4-yl}cyclopentanecarboxamide (22)

*Method A.* Scale: 0.96 mmol of compound **11.** Eluent for column chromatography was 2-5% MeOH in DCM. The product was recrystallized from methanol to give yellow crystals. Yield: 0.31 g (87%). MS (ESI) m/z: 365.9 [M+H]⁺¹. ¹H NMR (CDCl₃) *δ* 1.62-216 (m, 8H, 4CH₂), 2.80-2.97 (m, 1H, CH), 6.90 (bs, 1H, NH), 7.27-7.38 (m, 4H, Ar), 7.56-7.59 (m, 4H, Ar), 7.75-7.88 (m, 1H, Ar), 7.98 (s, 1H, NH). ¹³C NMR (CDCl₃) *δ* 25.96, 30.51, 47.34, 100.96, 116.30, 118.30, 120.85, 123.13, 127.31, 128.01, 129.01, 129.86, 138.80, 141.11, 147.93, 154.42, 175.25. Anal. calcd. for C₂₁H₂₀N₃OCl·0.3CH₂Cl₂ C, 66.29; H, 5.31; N, 10.74. Found C, 66.10; H, 5.28; N, 11.10.

### N-[2-(cyclopentylamino)quinolin-4-yl]cyclopentanecarboxamide (23)

*Method A.* Scale: 0.93 mmol of compound **11.** Eluent for column chromatography was 5% MeOH in DCM. The product was recrystallized from methanol to give off-white crystals. Yield: 0.039 g (14%). MS (ESI) m/z: 323.4 [M+H]⁺¹. ¹H NMR (CDCl₃) *δ* 1.40-2.16 (m, 16H, 8CH₂), 2.77-2.95 (m, 1H, CH), 4.15-4.32 (m, 1H, CH), 4.92 (bs, 1H, NH), 7.20 (t, 1H, *J*= 9.76 Hz, Ar), 7.48-7.84 (m, 6H, Ar, NH). ¹³C NMR (CDCl₃) *δ* 23.78, 25.96, 30.51, 33.64, 47.28, 53.20, 99.44, 115.54, 118.39, 121.49, 126.98, 129.58, 140.74, 148.72, 157.70, 175.28. Anal. calcd. for C₂₀H₂₅N₃O·0.7CH₂Cl₂ C, 67.13; H, 6.95; N, 10.97. Found C, 66.87; H, 7.23; N, 11.34.

### N-[2-(1H-indazol-6-ylamino)quinolin-4-yl]cyclopentanecarboxamide (24)

*Method A.* Scale: 0.87 mmol of compound **11.** Eluent for column chromatography was 5-10% MeOH in DCM. The product was recrystallized from methanol to give grey crystals. Yield: 0.15 g (47%). MS (ESI) m/z: 371.4 [M+H]⁺¹. ¹H NMR 300 MHz (CDCl₃+1 drop of MeOD) *δ* 1.62-2.03 (m, 8H, 4CH₂), 2.84-2.92 (m, 1H, CH), 7.26-7.32 (m, 2H, Ar), 7.43-7.50 (m, 2H, Ar), 7.58 (t, 1H, *J*= 5.33, 4.75 Hz, Ar), 7.67 (d, 1H, *J*= 5.33 Hz, Ar), 7.77 (d, 1H, *J*= 5.40 Hz, Ar), 7.85 (s, 1H, Ar), 8.02 (s, 1H, Ar), 8.08 (s, 1H, Ar). ¹³C NMR (CDCl₃+1 drop of MeOD) *δ* 25.84, 30.39, 46.46, 101.62, 110.57, 112.03, 116.85, 119.55, 122.37, 123.40, 126.52, 129.74, 133.25, 133.65, 137.41, 141.47, 147.87, 156.00, 176.31. Anal. calcd. for C₂₂H₂₁N₅O C, 71.13; H, 5.70; N, 18.86. Found C, 71.16; H, 5.75; N, 18.78.

### N-(2-anilinoquinolin-4-yl)cyclohexanecarboxamide (25)

*Method A.* Scale: 1.04 mmol of compound **12.** Eluent for column chromatography was 2-4% MeOH in DCM. The product was recrystallized from methanol to give white crystals. Yield: 0.26 g (73%). ¹H NMR (CDCl₃) *δ* 1.22-2.12 (m, 10H, 5CH₂), 2.31-2.49 (m, 1H, CH), 6.88 (bs, 1H, NH), 7.04 (t, 1H, *J =* 8.38, 7.62 Hz, Ar), 7.26-7.38 (m, 3H, Ar), 7.54-7.67 (m, 4H, Ar), 7.79 (d, 1H, *J*= 8.22 Hz, Ar), 7.87 (s, 1H, NH), 7.96 (s, 1H, Ar). ¹³C NMR (CDCl₃) *δ* 25.57, 25.84, 29.33, 44.16, 46.25, 98.56, 115.33, 120.40, 121.06, 122.28, 123.52, 125.19, 129.52, 131.31, 137.68, 141.23, 144.66, 153.91, 175.92. Anal. calcd. for C₂₂H₂₃N₃O·0.3H₂O C, 75.32; H, 6.78; N, 11.98. Found C, 75.14; H, 6.97; N, 11.88.

### N-{2-[(3,4-dichlorophenyl)amino]quinolin-4-yl}cyclohexanecarboxamide (26)

*Method B.* Scale: 0.17 mmol of compound **12.** Eluent for column chromatography was 1% MeOH in DCM. The product was recrystallized from ethyl acetate to give white crystals. Yield: 0.16 g (43%). ¹H NMR (CDCl₃) *δ* 1.23-2.17 (m, 10H, 5CH₂), 2.35-2.50 (m, 1H, CH), 6.77 (bs, 1H, NH), 7.32-7.41 (m, 2H, Ar), 7.49 (dd, 1H, *J*= 6.22, 2.56 Hz, Ar), 7.58-7.67 (m, 2H, Ar), 7.84-7.88 (m, 2H, Ar, NH), 7.95 (s, 1H, Ar), 8.09 (d, 1H, *J=* 2.56 Hz, Ar). ¹³C NMR (DMSO-d₆) *δ* 25.23, 25.48, 29.27, 38.30, 44.46, 103.69, 117.58, 118.24, 119.03, 121.55, 122.00, 122.55, 127.07, 129.67, 130.28, 130.80, 141.89, 141.98, 147.50, 154.26, 175.87. Anal. calcd. for C₂₂H₂₁Cl₂N₃O·0.5H₂O C, 62.42; H, 5.24; N, 9.93. Found C, 62.67; H, 5.20; N, 9.94.

### N-{2-[(4-methylphenyl)amino]quinolin-4-yl}cyclopentanecarboxamide (27)

*Method A.* Scale: 1.00 mmol of compound 12. Eluent DCM:ethylacetate:MeOH = 70:20:10. The product was recrystallized from MeOH:petroleum ether = 1:30. Yield: 0.14 g, 39%. ¹H NMR (CDCl₃) *δ* 1.30-2.09 (m, 10H, 5CH₂), 2.34-2.46 (m, 1H, CH), 2.34 (s, 3H, CH₃), 6.71 (bs, 1H, NH), 7.15-7.35 (m, 4H, Ar), 7.47-7.62 (m, 3H, Ar), 7.79 (d, 1H, *J* = 7.30 Hz, Ar), 7.99 (s, 1H, Ar). ¹³C NMR (DMSO-d*₆*) *δ* 20.58, 25.34, 29.46, 46.60, 100.64, 116.25, 118.20, 120.29, 122.32, 127.69, 129.42, 132.27, 137.27, 140.64, 148.25, 155.07, 174.60. Anal. calcd. for C₂₃H₂₅N₃O·0.5H₂O C, 74.97; H, 7.11; N, 11.40. Found C, 74.97; H, 6.96; N, 11.39.

### N-[2-(2,3-dihydro-1H-inden-5-ylamino)quinolin-4-yl]cyclohexanecarboxamide (28)

*Method A.* Scale: 1.20 mmol of compound **12.** Eluent 10-17% ethylacetate in DCM. The product was recrystallized from MeOH:petroleum ether = 1:30 to give white crystals. Yield: 0.08 g, 16%. ¹H NMR (CDCl₃) *δ* 1.35-2.40 (m, 10H, 5CH₂), 2.86-2.98 (m, 1H, CH), 6.72 (s, 1H, NH), 7.19-7.34 (m, 4H, Ar), 7.48-7.62 (m, 2H, Ar), 7.79 (d, 1H, *J =* 8.00 Hz, Ar), 7.97 (s, 1H, Ar). ¹³C NMR (DMSO-*d₆*) *δ* 14.16, 21.02, 25.58, 25.65, 29.70, 32.32, 33.08, 46.83, 60.37, 100.86, 116.43, 116.99, 118.42, 118.86, 122.48, 124.63, 127.83, 129.61, 138.11, 139.10, 140.81, 145.28, 148.45, 155.47, 174.78. Anal. calcd. for C₂₅H₂₇N₃O·2.4H₂O C, 71.17; H, 7.98; N, 9.09. Found C, 71.17; H, 7.63; N, 8.69.

### N-(2-anilinoquinolin-4-yl)benzamide (29)

*Method A.* Scale: 0.71 mmol of compound **13.** Eluent for column chromatography was 1-2% MeOH in DCM. The product was recrystallized from methanol to give white crystals. Yield: 0.12 g (49%). ¹H NMR (CDCl₃) *δ* 6.86 (bs, 1H, NH), 7.07 (t, 1H, *J*= 8.03, 6.57 Hz), 7.29-7.42 (m, 3H, Ar), 7.50-7.69 (m, 8H, Ar), 7.85 (d, 1H, *J*= 8.40 Hz, Ar), 7.93-7.98 (m, 1H, Ar), 8.11 (s, 1H, Ar), 8.50 (bs, 1H, NH). ¹³C NMR (DMSO-d*₆*) *δ* 107.03, 118.46, 118.88, 120.97, 122.19, 122.79, 126.79, 128.13, 128.55, 129.61, 132.04, 134.43, 141.65, 141.89, 148.02, 154.66, 166.663. Anal. calcd. for C₂₂H₁₇N₃O C, 77.86; H, 5.05; N, 12.38. Found C, 77.56; H, 5.20; N, 12.46.

### N-{2-[(3,4-dichloro-phenyl)amino]quinolin-4-yl}benzamide (30)

*Method B.* Scale: 0.94 mmol of compound **13.** Eluent for column chromatography was 0.25% MeOH in DCM. The product was recrystallized from methanol to give white crystals. Yield: 0.16 g (42%). ¹H NMR (CDCl₃) *δ* 6.85 (bs, 1H, NH), 7.39 (t, 2H, *J*= 8.40, 8.04 Hz, Ar), 7.49-7.71 (m, 6H, Ar), 7.87-7.99 (m, 3H, Ar), 8.09-8.13 (m, 2H, Ar), 8.54 (bs, 1H, NH). ¹³C NMR (DMSO-*d*₆) *δ* 106.45, 118.34, 118.85, 119.12, 121.76, 122.79, 126.95, 128.16, 128.52, 129.86, 130.37, 130.86, 132.10, 134.40, 141.77, 142.32, 147.59, 154.08, 166.75. Anal. calcd. for C₂₂H₁₅Cl₂N₃O C, 64.72; H, 3.70; N, 10.29. Found C, 64.42; H, 3.85; N, 10.34.

### N-(2-anilinoquinolin-4-yl)-2-furamide (31)

*Method A.* Scale: 0.58 mmol of compound **14.** Eluent for column chromatography was 1-2% MeOH in DCM. The product was recrystallized from methanol to give white crystals. Yield: 0.058 g (32%). ¹H NMR (CDCl₃) *δ* 6.52-6.54 (m, 1H, Ar), 7.02 (t, 1H, *J*= 7.30 Hz, Ar), 7.24-7.35 (m, 6H, Ar), 7.51-7.62 (m, 5H, Ar, NH), 8.09 (s, 1H Ar), 8.74 (bs, 1H, NH). ¹³C NMR (CDCl₃) *δ* 101.06, 112.74, 116.19, 118.22, 119.47, 122.19, 122.62, 127.74, 128.77, 129.47, 139.93, 140.17, 144.57, 147.02, 148.05, 154.63, 156.12. Anal. calcd. for C₂₀H₁₅N₃O₂ C, 72.93; H, 4.59; N, 12.75. Found C, 72.35; H, 4.61; N, 12.59.

### N-{2-[(3,4-dichlorophenyl)amino]quinolin-4-yl}-2-furamide (32)

*Method B.* Scale: 0.77 mmol of compound **14.** Eluent for column chromatography was 0-1% MeOH in DCM. The product was recrystallized from methanol to give white crystals. Yield: 0.18 g (59%). ¹H NMR (CDCl₃) *δ* 6.58-6.61 (m, 1H, Ar), 7.26-7.50 (m, 4H, Ar), 7.59-7.83 (m, 4H, Ar), 8.08 (s, 1H, Ar), 8.15 (s, 1H, Ar). ¹³C NMR (DMSO-*d*₆) *δ* 106.42, 112.36, 115.88, 118.37, 118.64, 119.19, 121.82, 122.61, 122.85, 126.95, 129.92, 130.34, 130.86, 141.50, 141.71, 146.44, 146.95, 147.56, 153.99, 156.96. Anal. calcd. for C₂₀H₁₃Cl₂N₃O₂ C, 60.23; H, 3.29; N, 10.55. Found C, 60.10; H, 3.48; N, 10.59.

### N-(2-anilinoquinolin-4-yl)cyclobutanecarboxamide (33)

*Method B.* Scale: 0.77 mmol of compound **15.** Eluent for column chromatography was 1-2% MeOH in DCM. The product was recrystallized from ethyl acetate to give white crystals. Yield: 0.17 g (71%). ¹H NMR (CDCl₃) *δ* 1.93-2.56 (m, 6H, 3CH₂), 3.24-3.41 (m, 1H, CH), 6.84 (bs, 1H, NH), 7.05 (t, 1H, *J* = 7.30 Hz, Ar), 7.26-7.40 (m, 3H, Ar), 7.53-7.82 (m, 6H, Ar, NH), 7.99 (s, 1H, NH). ¹³C NMR (CDCl₃) *δ* 17.75, 25.11, 40.64, 101.94, 116.52, 118.74, 119.40, 122.10, 122.38, 127.41, 128.74, 129.35, 140.32, 140.50, 147.90, 154.63, 174.28. Anal. calcd. for C₂₀H₁₉N₃O·0.5H₂O C, 73.60; H, 6.18; N, 12.87. Found C, 73.94; H, 6.15; N, 12.95.

### N-{2-[(3,4-dichlorophenyl)amino]quinolin-4,yl}cyclobutanecarboxamide (34)

*Method B.* Scale: 0.77 mmol of compound **15.** Eluent for column chromatography was 0-0.5% MeOH in DCM. The product was recrystallized from methanol to give white crystals. Yield: 0.13 g (44%). ¹H NMR (CDCl₃) *δ* 1.95-2.53 (m, 6H, 3CH₂), 3.23-3.42 (m, 1H, CH), 6.79 (bs, 1H, NH), 7.31-7.39 (m, 2H, Ar), 7.47-7.70 (m, 5H, Ar, NH), 7.86 (d, 1H, *J*= 8.40 Hz, Ar), 7.95 (s, 1H, Ar), 8.09 (s, 1H, Ar). ¹³C NMR (DMSO-*d*₆) *δ* 17.90, 24.78, 103.57, 117.49, 118.25, 119.06, 121.58, 121.94, 122.58, 127.10, 129.70, 130.28, 130.80, 141.89, 141.95, 147.47, 154.29, 174.45. Anal. calcd. for C₂₀H₁₇Cl₂N₃O C, 62.19; H, 4.44; N, 10.88. Found C, 61.85; H, 4.65; N, 10.82.

Table 1 summarizes the chemical structures and physico-chemical characteristics of the 2,4-disubstituted quinoline derivatives prepared as described above.

**Table 1: Chemical structures and physico-chemical characteristics**

| Nr | Compound | Molecular Formula | MW | Mp (°C) | MS | Recrystallization solvent |
|---|---|---|---|---|---|---|
| 16 | 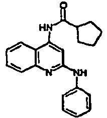 | C₂₁H₂₁N₃O | 331.42 | 173-174 | 332.2 | MeOH |
| 17 | 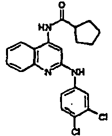 | C₂₁H₁₉N₃OCl₂ | 400.31 | 216-217 | 400.2 | MeOH |
| 18 | 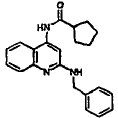 | C₂₂H₂₃N₃O | 345.45 | 154-155 | 345.9 | MeOH |
| 19 | 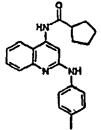 | C₂₂H₂₃N₃O | 345.45 | 188-190 | 345.5 | MeOH |
| 20 | 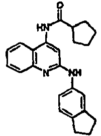 | C₂₄H₂₅N₃O | 371.49 | 115-117 | 372.2 | MeOH |
| 21 | 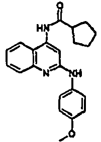 | C₂₂H₂₃N₃O₂ | 361.45 | 191-192 | 362.1 | MeOH |
| 22 | 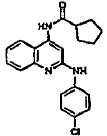 | C₂₁H₂₀N₃OCl | 365.87 | 198-199 | 366.1 | MeOH |
| 23 | 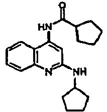 | C₂₀H₂₅N₃O | 323.44 | 183-184 | 324.0 | MeOH |
| 24 | 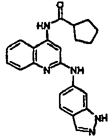 | C₂₂H₂₁N₅O | 371.45 | 222-224 | 371.9 | MeOH |
| 25 | 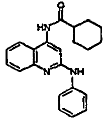 | C₂₂H₂₃N₃O | 345.45 | 191-192 | - | MeOH |
| 26 | 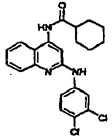 | C₂₂H₂₁Cl₂N₃O | 314.34 | 226-227 | - | EA |
| 27 | 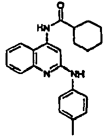 | C₂₃H₂₅N₃O | 359.20 | 189-190 | 360.27 | MeOH/PE |
| 28 | 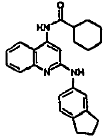 | C₂₅H₂₇N₃O | 385.22 | 175-176 | 386.27 | MeOH/PE |
| 29 | 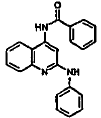 | C₂₂H₁₇N₃O | 339.40 | 223-224 | - | MeOH |
| 30 | 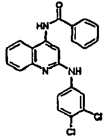 | C₂₂H₁₅Cl₂N₃O | 408.29 | 224-225 | - | MeOH |
| 31 | 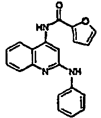 | C₂₀H₁₅N₃O₂ | 329.36 | 215-216 | - | MeOH |
| 32 | 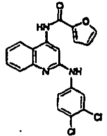 | C₂₀H₁₃Cl₂N₃O₂ | 398.25 | 217-218 | - | MeOH |
| 33 | 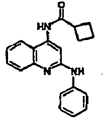 | C₂₀H₁₉N₃O | 317.39 | 154-155 | - | EA |
| 34 | 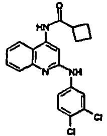 | C₂₀H₁₇Cl₂N₃O | 386.28 | 193-194 | - | MeOH |

| | | | | | | |
|---|---|---|---|---|---|---|
| MW: molecular weight (D); Mp: melting point (°C); MS: mass spectrometry data (M+H) | | | | | | |

### Biological Experiments

### Materials:

[¹²⁵I]*N⁶*-(4-amino-3-iodobenzyl)adenosine-5'-*N*-methyluronamide (I-AB-MECA; 2000 Ci/mmol), was from Amersham Pharmacia Biotech (Buckinghamshire, UK).

### Cell culture and membrane preparation:

CHO (Chinese hamster ovary) cells expressing the recombinant human A₃ receptors were cultured in DMEM and F12 (1:1) supplemented with 10% fetal bovine serum, 100 units/ml penicillin, 100 µg/ml streptomycin and 2 µmol/ml glutamine. Cells were harvested by trypsinization. After homogenization and suspension, cells were centrifuged at 500 g for 10 min, and the pellet was re-suspended in 50 mM Tris·HCl buffer (pH 7.4) containing 10 mM MgCl₂. The suspension was homogenized with an electric homogenizer for 10 sec, and was then re-centrifuged at 20,000 g for 20 min at 4°C. The resultant pellets were resuspended in buffer in the presence of 3 Units/mL adenosine deaminase, and the suspension was stored at -80°C until the binding experiments. The protein concentration was measured using the Bradford assay.

### Binding Assay to the Human A₃ AR:

Each tube in the competitive binding assay contained 100 µl membrane suspension (20 µg protein), 50 µl [¹²⁵I]I-AB-MECA (0.5 nM), and 50 µl of increasing concentrations of the test modulators in Tris·HCl buffer (50 mM, pH 8.0) containing 10 mM MgCl₂, 1 mM EDTA. Nonspecific binding was determined using 10 µM of 5'-*N*-ethylcarboxamidoadenosine in the buffer. The mixtures were incubated at 25°C for 60 min. Binding reactions were terminated by filtration through Whatman GF/B filters under reduced pressure using a MT-24 cell harvester (Brandell, Gaithersburgh, MD, USA). Filters were washed three times with 9 mL ice-cold buffer. Radioactivity was determined in a Beckman 5500B γ-counter.

### Dissociation kinetics of [¹²⁵I]I-AB-MECA from human A₃ARs:

The dissociation of [¹²⁵I]I-AB-MECA was measured as follows. Membranes (20 µg) were pre-incubated at 25°C with 0.5 nM [¹²⁵I]I-AB-MECA, in a total volume of 100 µl of Tris-HCl buffer (50 mM, pH 8.0) containing 10 mM MgCl₂, and 1 mM EDTA for 60 min. The dissociation was then initiated by the addition of 3 µM Cl-IB-MECA with or without allosteric modulators. The time course of dissociation of total binding was measured by rapid filtration at appropriate time intervals. Nonspecific binding was measured after 60-min incubation in the presence of 3 µM Cl-IB-MECA. Further assay was as described above.

### Statistical analysis

Binding parameters were calculated using Prism 5.0 software (GraphPAD, San Diego, CA, USA). IC₅₀ values obtained from competition curves were converted to Kᵢ values using the Cheng-Prusoff equation. Data were expressed as mean ± standard error.

**Table 2: Potency of N-[(2-amino)quinolin-4-yl]carboxamide derivatives in binding assays at human A₃ARs and allosteric effects at the human A₃AR**

| ***No.*** | ***R₁*** | ***R₂*** | ***% displacement at 10µM (hA₃AR)^{a}*** | ***hA₃ARAg*** |
|---|---|---|---|---|
| | | | | ***Dissociation*^{b} *at 10 µM*** |
| **16** | *c*-pent | Ph | 61 (56/66) | 145 (142/147) |
| **17** | *c*-pent | 3,4-Cl₂-Ph | 50 (45/55) | 169 (168/170) |
| **18** | *c*-pent | PhCH₂ | 28 (23/34) | 107 (105/108) |
| **19** | *c*-pent | 4-Me-Ph | 63 (61/64) | 165 (160/170) |
| **20** | *c*-pent | 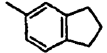 | 62 (60/64) | 147 (143/150) |
| **21** | *c*-pent | 4-OMe-Ph | 35 (24/45) | 134 (131/137) |
| **22** | *c*-pent | 4-Cl-Ph | 28 (18/38) | 171 (169/173) |
| **23** | *c*-pent | *c*-pent | 14 (9/19) | 114 (113/114) |
| **24** | *c*-pent | 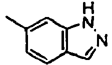 | 90 (88/91) | 102 (101/102) |
| **25** | *c*-hex | Ph | 26 (24/28) | 210 (205/214) |
| **26** | *c*-hex | 3,4-Cl₂-Ph | 17 (16/17) | 249 (264/234) |
| **27** | *c*-hex | 4-Me-Ph | 64 (63/65) | 220 (228/211) |
| **28** | *c*-hex | 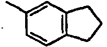 | 42 (37/46) | 217 (213/221) |
| **29** | Ph | Ph | 76 (76/76) | 100 (98/101) |
| **30** | Ph | 3,4-Cl₂-Ph | 37 (36/38) | 112 (106/117) |
| **31** | 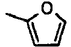 | Ph | 60 (60/60) | 100 (98/101) |
| **32** | 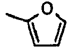 | 3,4-Cl₂-Ph | 13 (7/18) | 96 (93/99) |
| **33** | *c*-but | Ph | 66 (64/69) | 119 (116/122) |
| **34** | *c*-but | 3,4-Cl₂-Ph | 51 (49/52) | 149 (149/149) |

| | | | | |
|---|---|---|---|---|
| a) All experiments were performed using adherent CHO (A₃), cells stably transfected with cDNA encoding the human ARs. Binding at human A₃ARs in this study was carried out as described in Methods using [¹²⁵I]I-AB-MECA (0.1 nM) as a radioligand. Values from the present study are expressed as mean ± s.e.m., n = 3-5. Percentage inhibition at A₃ receptors is expressed as the mean value from 2-4 separate experiments with similar results performed in duplicate. b) Dissociation by Cl-AB-MECA in the absence of modulator (control) was set at 100%. Increase in binding of control was determined after 1.5 or 2 hours of dissociation by Cl-AB-MECA in presence of 10 µM of the test compounds, respectively. Values are means of two separate assays performed in duplicate (within brackets are the result of each of the two assays). | | | | |

In **Table 2** the effects of the 2,4-disubstituted quinoline derivatives at the orthosteric site of the human adenosine A₃ receptor are listed (column 4), together with their effects on the allosteric site on the human adenosine A₃ receptor (column 5). Many compounds display little if any affinity for the orthosteric binding site, especially when R₁ = 3,4-Cl₂-phenyl (≤50% displacement). The best separation between orthosteric and allosteric recognition was found with compound **26** with little displacement at the orthosteric site (17%) and a huge allosteric effect (247% vs the control value of 100%). Most compounds significantly retard the dissociation of the radioligand.

## Claims

1. An As adenosine receptor allosteric modulator (AsRM, having the following general formula (I): 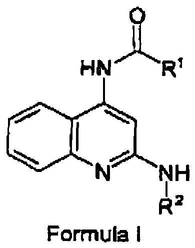 wherein:
R₁ is a group selected from C₄-C₁₂ cycloalkyl, C₄-C₁₂ cycloalkenyl, C₆-C₁₂ aryl, C₄-C₁₂ heteroaryl, alkcycloalkyl, alkaryl, C₁-C₁₀ alkyl, C₂-C₁₀ alkenyl; C₂-C₁₀ alkynyl, C₅-C₁₆ fused cycloalkyl, bicyclic aromatic or heteroaromatic ring; C₁-C₁₀ alkylether, amino, hydrazido, C₁-C₁₀ alkylamino, C₁-C₁₀ alkoxy, C₁-C₁₀ alkoxycarbony, C₁-C₁₀ alkanal, C₁-C₁₀acyl, C₁-C₁₀ thioalkoxy, pyridylthio, thio, and C₁-C₁₀ alkylthio, acetoamido and subtonic acid;
R₂ is a group selected from hydrogen or a cyclic moiety selected from the group consisting of aryl, heteroaryl, alkaryl, alkheteroaryl, cycloalkyl, heterocycloalkyl, cycloalkenyl, heterocycloalkenyl, cycloalkynyl, heterocycloalkynyl, alkcycloalkyl, alkcycloheteroalkyl, alkcycloalkenyl and alkheterocycloalkenyl said cyclic moiety optionally substituted by at least one group selected from C₁-C₁₀ alkyl, halo, C₁.C₁₀ alkanol, hydroxyl, C₁-C₁₀ acyl, C₁-C₁₀, alkoxy; C₁-C₁₀-alkoxycarbony, C₁-C₁₀ alkoxylalkyl; C₁-C₁₀ thioalkoxy; C₁-C₁₀ alkylether, amino, hydrazido, C₁-C₁₀ alkylamino, pyridylthio, C₂-C₁₀ alkenyl; C₂-C₁₀ alkynyl, thio, C₁-C₁₀ alkylthio, acetoamido and sulfonic acid; or said substituents can form together with an atom of a cyclic moiety a cycloalkyl, heterocycloalkyl, cycloalkenyl or heterocycloalkenyl fused to said cyclic moiety;
and pharmaceutically acceptable salts thereof,
for use in the treatment of a condition which is treatable by a compound selected from the group consisting of adenosine, an As adenosine receptor agonist or an As adenosine receptor antagonist, wherein said condition is selected from immuno-compromised afflictions, hyperproliferative disorder, benign hyperplasic disorder, skin proliferative disease, inflammatory disease, ischemic conditions, and conditions associated with high intraocular pressure.

2. The AsRM for use according to Claim 1, wherein R¹ is selected from cycloalkyl, aryl and heteroaryl.

3. The AsRM for use according to Claim 1 or 2, wherein R² is selected from aryl, alkaryl, cycloalkyl, the aryl or cycloalkyl being optionally substituted by at least one substituent selected from C₁. C₁₀ alkyl, halo and C₁-C₁₀ alkylether.

4. The A₃RM for use according to any one of Claims 1 to 3, wherein R¹ is selected from
C₄-C₆ cycloalkyl, phenyl or a five membered heterocyclic aromatic ring having the following formula (II): 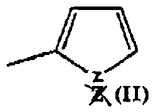 wherein Z is selected from 0, S or NH; and
R² is selected from C₄-C₆ cycloalkyl, phenyl, alkphenyl, or an aromatic ring fused to a five membered cyclic or heteroaromatic ring having the following formulae (IIIa) or (IIIb): 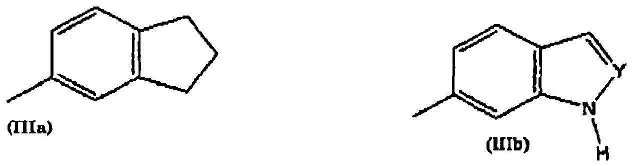 wherein Y is selected from N or CH;
the aryl or cycloalkyl ring in said cycloalkyl, phenyl, alkphenyl or in formulae (Va) or (Vb) being optionally substituted with a substituent selected from C₁-C₁₀ alkyl, halo, or C₁-C₁₀ alkylether.

5. The A₉RM for use according to any one of Claims 1 to 4, being a 2,4-disubstituted quinoline derivative selected from:
N-(2-anilinoquinolin-4-yl)cyclopentanecarboxamide
N-{2-[(3,4-dichlorophenyl)amino]quinolin-4-yl}cyclopentanecarboxamide
N-[2-(benzylamino)quinolin-4-yl]cyclopentanecarboxamide
N-{2-[(4-methylphenyl)amino]quinolin-4-yl}cyclopentanecarboxamide
N-[2-(2,3-dihydro-1H-inden-5-ylamino)quinolin-4-yl] cyclopentanecarboxamide
N-{2-[(4-methoxyphenyl)amino]quinolin-4-yl}cyclopentanecarboxamide
N-{2-[(4-chlorophenyl)amino]quinolin-4-yl}cyclopentanecarboxamide
N-[2-(cyclopentylamino)quinolin-4-yl]cyclopentanecarboxamide
N-[2-(1H-indazol-6-ylamino)quinolin-4-yl]cyclopentanecarboxamide
N-(2-anilinoquinolin-4-yl)cyclohexanecarboxamide
N-{2-[(3,4-dichlorophenyl)amino]quinolin-4-yl}cyclohexanecarboxamide
N-{2-[(4-methylphenyl)amino]quinolin-4-yl}cyclohexanecarboxamide
N-[2-(2,3-dihydro-1H-inden-5-ylamino)quinolin-4-yl] cyclohexanecarboxamide
N-(2-anilinoquinolin-4-yl)benzamide
N-{2-[(3,4-dichloro-phenyl)amino]quinolin-4-yl}benzamide
N-(2-anilinoquinolin-4-yl)-2-furamide
N-{2-[(3,4-dichlorophenyl)amino]quinolan-4-yl}-2-furamide
N-(2-anilinoquinolin-4-yl)cyclobutanecarboxamide
N-{2-[(3,4-dichlorophenyl)amino]quinolin-4-yl}cyclobutanecarboxamide.

6. The A₃RM for use according to any one of Clams 1 to 5, whereby said use is for enhancing the activity of an A₃ adenosine receptor (A₃AR) thereby treating a condition treatable by adenosine or an A₃AR agonist, wherein said condition is selected from hyperproliferative disorder, benign hyperplasic disorder, skin proliferative disease, inflammatory disease, ischemic conditions, and conditions associated with high intraocular pressure.

7. The A₃RM for use according to Claim 6 wherein said R¹ is a C₄-C₆ cycloalkyl or a phenyl; and
R² is selected from C₄-C₆ cycloalkyl, phenyl or an aromatic ring fused to a five membered cycloalkyl having the following formulae (III): 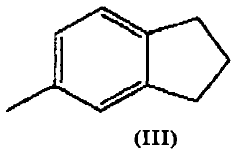 the phenyl moiety in R² being unsubstituted or substituted at least once with a C₁-C₃ alkyl, halogen or C₁-C₃ alkether.

8. The A₃RM for use according to Claim 7, wherein said R¹ is selected from cyclopentyl, cyclohexyl, cyclo butyl or phenyl; and
R² is selected from cyclopentyl, phenyl or an aromatic ring fused to a five membered cycloalkyl having the following formulae (III): 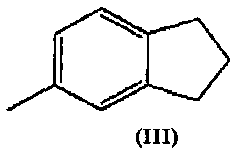 the phenyl moiety in R² being unsubstituted or substituted at least once with a methyl, Cl or methylether.

9. The A₃RM for use according to Claim 8, being a 2,4.disubstituted quinoline derivative selected from:
N-{2-[(3,4-dichlorophenyl)amino]quinolin-4-yl}cyclopontanecarboxamide
N-{2-[(4-methoxyphonyl)amino]quinolin-4-yl}cyclopentanocarboxamide
N-{2-[(4-chlorophenyl)amino]quinolin-4-yl}cyclopentanecarboxamide
N-(2-anilinoquinolin-4-yl)cyclohexanecarboxamide
N-{2-[(3,4-dichlorophenyl)amino]quinolin-4-yl}cyclohexanecarboxamide
N-{2-[(4-methylphenyl)amino]quinolin-4-yl}cyclohexanecarboxamide
N-[2-(2,3-dihydro-1H-inden-5-ylamino)quinolin-4-yl] cyclohexanecarboxamide
N-{2-[(3,4-dichlorophenyl)amino]quinolin-4-yl)cyclobutanecarboxamide.

10. The A₃RM for use according to Claim 8, being selected from
N-{2-[(4-chlorophenyl)amino]quinolin-4-yl}cyclopentanecarboxamide
N-(2-anilinoquinolin-4-yl)cyclohexanecarboxamide
N-{2-[(3 4-dichlorophenyl)amino]quinolin-4-yl}cyclohexanecarboxamide
N-[2-(2,3-dihydro-1H-inden-5-ylamino)quinolin-4-yl] cyclohexanecarboxamide

11. The A₃RM for use according to Claim 10, being N-{2-[(3,4-dichlorophenyl) amino]quinolin-4-yl}cyclohexanecarboxamide.

12. The A₃RM for use according to any one of Claims 6 to 9, being an A₃AR enhancer for enhancing the activity of the A₃AR, the enhancement comprises occurrence of one or more of the following:
(a) an increase of at least 15% in efficacy of said A₃AR by binding of said A3AR enhancer to an allosteric site of said A₃AR; or
(b) a decrease in dissociation rate of adenosine or an AJAR agonist from its binding site.

13. A 2,4-disubstituted quinoline derivative selected from the group consisting of:
N-(2-anilinoquinolin-4-yl)cyclopentanecarboxamide
N-{2-[{3,4-dichloxophenyl)amino]quinolin-4-yl}cyclopentanecarboxamide
N-[2-(benzylamino)quinolin-4-yl]cyclopentanecarboxamide
N-{2-[(4-methylphenyl)amino]quinolin-4-yl)cyclopentanecarboxamide
N-[2-(2,3-dihydro-1H-inden-5-ylamino)quinolin-4-yl] cyclopentanecarboxamide
N-{2-[(4-methoxyphenyl)amino]quinolin-4-yl}eyclopentanocarboxamide
N-{2-[(4-chlorophenyl)amino]quinolin-4-yl}cyclopentanocarboxamide
N-[2-(cyclopentylamino)quinolin-4-yl]cyclopentanecarboxamide
N-[2-(1H-indazol-6-ylamino)quinolin-4-yl]cyclopentanecarboxamide
N-(2-anilinoquinolin-4-yl)cyclohexanecarboxamide
N-{2-[(3,4-dichlorophenyl)amino]quinolin-4-yl}cyclohexanecarboxamide
N-{2-[(4-methylphenyl)amino]quinolin-4-yl}cyclohexanecarboxamide
N-[2-{2,3-dihydro-1H-inden-5-ylamino)quinolin-4-yl] cyclohexanecarboxamide
N-(2-anilinoquinolin-4-yl)benzamide
N-{2-[(3,4-dichloro-phenyl)amino]quinolin-4-yl}benzamide
N-(2-anilinoquinolin-4-yl)-2-furamide
N-{2-[(3,4-dichlorophenyl)amino]quinolin-4-yl}-2-furamide
N-(2-anilinoquinolin-4-yl)cyclobutanecarboxamide; and
N-{2-[(3,4-dichlorophenyl)amino]quinolin-4-yl}cyclobutanecarboxamide.

14. The 2,4-disubstituted quinoline derivative of Claim 13, selected from the group consisting of:
N-{2-[(3,4-dichlorophenyl)amino]quinolin-4-yl}cyclopentanecarboxamide
N-{2-[(4-methoxyphenyl)amino]quinolin-4-yl}cyclopentanecarboxamide
N-{2-[(4-chlorophonyl)amino] quinolin-4-yl}cyclopentanecarboxamide
N-(2-anilinoquinolin-4-yl)cyclohexanecarboxamide
N-{2-[(3,4-dichlorophenyl)amino]quinolin-4-yl}cyclohexanecaxboxamide
N-{2-[(4-methylphenyl)amino]quinolin-4-yl}cyclohexanecarboxamide
N-[2-(2,3-dihydro-1H-inden-5-ylamino)quinolin-4-yl] cyclohexanecarboxamide, and
N-{2-[(3,4-dichlorophenyl)amino]quinolin-4-yl}cyclobutanecarboxamide.

15. The 2,4-disubstituted quinoline derivative of Claim 14, selected from the group consisting of:
N-{2-[(4-chlorophenyl)amino]quinolin-4-yl}cyclopentanecarboxamide
N-(2-anilinoquinolin-4-yl)cyclohexanecarboxamide
N-{2-[(3,4-dichlorophenyl)amino]quinolin-4-yl}cyclohexanecarboxamide
N-[2-(2,3-dihydro-1H-inden-5-ylamino)quinolin-4-yl] cyclohexanecarboxamide.

## Patentansprüche

1. A₃ Adenosinrezeptor allosterischer Modulator (A₃RM) mit folgender allgemeiner Formel (I): 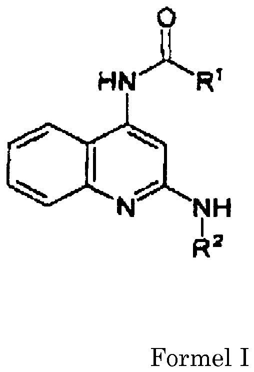 wobei:
R₁ eine Gruppe ist, ausgewählt aus C₄-C₁₂ Cycloalkyl, C₄-C₁₂ Cyucloalkenyl, C₈-C₁₂ Aryl, C₄-C₁₂ Heteroaryl, Alkcycloalkyl, Alkaryl, C₁-C₁₀ Alkyl, C₂-C₁₀ Alkenyl; C₂-C₁₀ Alkynyl, C₅-C₁₅ geschmolzenem Cycloalkyl, bizyklischem aromatischem oder heteroaromatischem Ring; C₁-C₁₀ Alkylether, Amin, Hydrazid, C₁-C₁₀ Alkylamin, C₁-C₁₀ Alkoxy, C₁-C₁₀ Alkoxycarbon, C₁-C₁₀ Alkanol, C₁-C₁₀ Acyl, C₁-C₁₀ Thioalkoxy, Pyridylthio, Thio und C₁-C₁₀ Alkylthio, Acetamid und Sulfonsäure;
R2 eine Gruppe ist, ausgewählt aus Wasserstoff oder einem zyklischen Rest, ausgewählt aus der Gruppe bestehend aus Aryl, Heteroaryl, Alkaryl, Alkheteroaryl, Cycloalkyl, Heterocycloalkyl, Cycloalkenyl, Heterocycloalkenyl, Cycloalkynyl, Heterocycloalkynyl, Alkcycloalkyl, Alkcycloheteroalkyl, Alkcycloalkenyl und Alkheterocycloalkenyl, wobei der zyklische Rest optional substituiert ist durch mindestens eine Gruppe ausgewählt aus C₁-C₁₀ Alkyl, Halogen, C₁-C₁₀ Alkanol, Hydroxyl, C₁-C₁₀ Acyl, C₁-C₁₀ Alkoxy, C₁-C₁₀ Alkoxycarbon, C₁-C_{10.} Alkoxylalkyl; C₁-C₁₀ Thioalkoxy; C₁-C₁₀ Alkylether, Amin, Hydrazid, C₁-C₁₀Alkylamin, Pyridylthio, C₂-C₁₀ Alkenyl; C₂-C₁₀ Alkynyl, Thio, C₁-C₁₀ Alkylthio, Acetamid und Sulfonsäure; oder die Substituenten können zusammen mit einem Atom eines zyklischen Rests ein Cycloalkyl, Heterocycloalkyl, Cycloalkenyl oder Heterocycloalkenyl, verschmolzen zu dem zyklischen Rest, bilden;
und pharmazeutisch akzeptablen Salzen davon,
zur Verwendung bei der Behandlung eines Zustands, der durch eine Verbindung therapierbar ist, ausgewählt aus der Gruppe bestehend aus Adenosin, einem A₃ Adenosinrezeptoragonisten oder einem A₃ Adenosinrezeptorantagonisten, wobei der Zustand ausgewählt ist aus immunsupprimierten Leiden, hyperproliferativen Krankheiten, gutartigen hyperplasischen Krankheiten, hautproliferativen Krankheiten, Entzündungskrankheiten, ischämischen Zuständen und Zuständen im Zusammenhang mit hohem Augeninnendruck.

2. A₃RM zur Verwendung nach Anspruch 1, wobei R¹ ausgewählt ist aus Cycloalkyl, Aryl und Heteroaryl.

3. A₃RM zur Verwendung nach Anspruch 1 oder 2, wobei R² ausgewählt ist aus Aryl, Alkaryl, Cycloalkyl, wobei Aryl oder Cycloalkyl optional substituiert sind durch mindestens einen Substituenten, ausgewählt aus C₁-C₁₀ Alkyl, Halogen und C₁-C₁₀ Alkylether.

4. A₃RM zur Verwendung nach einem der Ansprüche 1 bis 3, wobei R¹ ausgewählt ist aus C₄-C₆ Cycloalkyl, Phenyl oder einem fünfgliedrigen heterozyklischen aromatischen Ring folgender Formel (II): 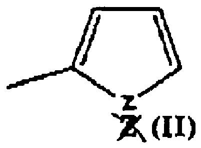 wobei Z ausgewählt ist aus 0, S oder NH; und
R² ausgewählt ist aus C₄-C₆ Cycloalkyl, Phenyl, Alkylphenyl oder einem aromatischen Ring, geschmolzen auf einen fünfgliedrigen zyklischen oder heteroaromatischen Ring mit folgender Formel (IIIa) oder (IIIb): 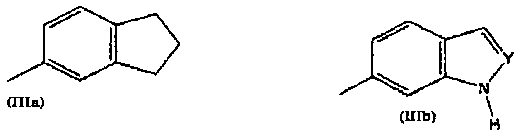 Wobei Y ausgewählt ist aus N oder CH; der Aryl- oder Cycloalkylring in dem Cycloalkyl, Phenyl, Alkphenyl oder in den Formeln (Va) oder (Vb) optional substituiert sind mit einem Substituenten, ausgewählt aus C₁-C₁₀ Alkyl, Halogen oder C₁-C₁₀ Alkylether.

5. A₃RM zur Verwendung nach einem der Ansprüche 1 bis 4, der ein 2,4-disubstituiertes Quinolinderivat ist, ausgewählt aus:
N-(2-Anilinquinolin-4-yl)cyclopentancarboxamid
N-(2- [(3,4-Dichlorphenyl)amin] quinolin-4-yl)cyclopentancarboxamid
N-[2-(Benzylamin)quinolin-4-yl]cyclopentancarboxamid
N-(2-[(4-Methylphenyl)amin]quinolin-4-yl)cyclopentancarboxamid
N-[2-(2,3-Dihydro-1H-inden-5-ylamin)quinolin-4-yl] cyclopentancarboxamid
N-(2-[(4-Methoxyphenyl)amin]quinolin-4-yl)cyclopentancarboxamid
N-(2-[(4-Chlorphenyl)amin]quinolin-4-yl)cyclopentancarboxamid
N-[2-(Cyclopentylamin)quinolin-4-yl]cyclopentancarboxamid
N-[2-(1H-indazol-6-ylamin)quinolin-4-yl]cyclopentancarboxamid
N-(2-Anilinquinolin-4-yl)cyclohexancarboxamid
N-(2-[(3,4-Dichlorphenyl)amin]quinolin-4-yl)cyclohexancarboxamid
N-(2-[(4-Methylphenyl)amin]quinolin-4-yl)cyclohexancarboxamid
N-[2-(2,3-Dihydro-1H-inden-5-ylamin)quinolin-4-yl]cyclohexancarboxamid
N-(2-Anilinquinolin-4-yl)benzamid
N-(2-[(3,4-Dichlorphenyl)amin]quinolin-4-yl]benzamid
N-(2-Anilinquinolin-4-yl)-2-furamid
N-(2-[(3,4-Dichlorphenyl)amin]quinolin-4-yl]-2-furamid
N-(2-Anilinquinolin-4-yl)cyclobutancarboxamid
N-(2-[(3,4-Dichlorphenyl)amin]quinolin-4-yl)cyclobutancarboxamid.

6. A₃RM zur Verwendung nach einem der Ansprüche 1 bis 5, wobei die Verwendung zur Verbesserung der Aktivität eines A₃ Adenosinrezeptors (A₃AR) ist, wodurch ein Zustand behandelt wird, der durch Adenosin oder einen A₃AR-Agonisten behandelbar ist, wobei der Zustand ausgewählt ist aus hyperproliferativen Krankheiten, gutartigen hyperplasischen Krankheiten, hautproliferativen Krankheiten, Entzündungskrankheiten, ischämischen Zuständen und Zuständen im Zusammenhang mit hohem Augeninnendruck.

7. A₃RM zur Verwendung nach Anspruch 6, wobei R¹ ein C₄-C₆ Cycloalkyl oder ein Phenyl ist; und
R² ausgewählt ist aus C₄-C₆ Cycloalkyl, Phenyl oder einem aromatischen Ring, geschmolzen auf ein fünfgliedriges Cycloalkyl der folgenden Formel (III): 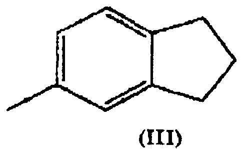 wobei der Phenylrest in R² nicht subsituiert ist oder mindestens einmal mit einem C₁-C₃ Alkyl, Halogen oder C₁-C₃ Alkether substituiert ist.

8. A₃RM zur Verwendung nach Anspruch 7, wobei R1 ausgewählt ist aus Cyclopentyl, Cyclohexyl, Cyclobutyl oder Phenyl; und
R² ausgewählt ist aus Cyclopentyl, Phenyl oder einem aromatischen Ring, geschmolzen auf ein fünfgliedriges Cycloalkyl der folgenden Formel (III): 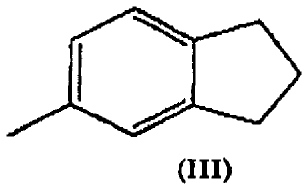 wobei der Phenylrest in R² nicht substituiert ist oder mindestens einmal mit einem Methyl, Cl oder Methylether substituiert ist.

9. A₃RM zur Verwendung nach Anspruch 8, der ein 2,4-disubstituiertes Quinolinderivat ist, ausgewählt aus:
N-(2- [(3,4-Dichlorphenyl)amin] quinolin-4-yl)cyclopentancarboxamid
N-(2- [(4-Methoxyphenyl)amin] quinolin-4-yl)cyclopentancarboxamid
N-(2- [(4-Chlorphenyl)amin] quinolin-4-yl)cyclopentancarboxamid
N-(2-Anilinquinolin-4-yl)cyclohexancarboxamid
N-(2- [(3,4-Dichlorphenyl)amin] quinolin-4-yl)cyclohexancarboxamid
N-(2- [(4-Methylphenyl)amin] quinolin-4-yl)cyclohexancarboxamid
N-[2-(2,3-Dihydro-1H-inden-5-ylamin)quinolin-4-yl]cyclohexancarboxamid
N-(2- [(3,4-Dichlorphenyl)amin] quinolin-4-yl)cyclobutancarboxamid

10. A₃RM zur Verwendung nach Anspruch 8, ausgewählt aus:
N-(2- [(4-Chlorphenyl)amin] quinolin-4-yl)cyclopentancarboxamid
N-(2-Anilinquinolin-4-yl)cyclohexancarboxamid
N-(2- [(3,4-Dichlorphenyl)amin] quinolin-4-yl)cyclohexancarboxamid
N-[2-(2,3-Dihydro-1H-inden-5-ylamin)quinolin-4-yl]cyclohexancarboxamid

11. A₃RM zur Verwendung nach Anspruch 10, der N-(2-[(3,4-Dichlorphenyl)amin] quinolin-4-yl)cyclohexancarboxamid ist.

12. A₃RM zur Verwendung nach einem der Ansprüche 6 bis 9, der ein A₃AR Verbesserer zur Verbesserung der Aktivität des A₃AR ist, wobei die Verbesserung das Vorkommen von einem oder mehr der Folgenden umfasst:
(a) eine Zunahme von mindestens 15 % an Wirksamkeit des A₃AR durch Bindung des A₃AR Verbesserers an eine allosterisches Stelle des A₃AR; oder
(b) eine Verminderung des Dissoziationsrate von Adenosin oder einem A₃AR Agonisten von dessen Bindungsstelle.

13. 2,4-disubstituiertes Quinolinderivat, ausgewählt aus der Gruppe bestehend aus:
N-(2-Anilinquinolin-4-yl)cyclopentancarboxamid
N-(2-[(3,4-Dichlorphenyl)amin]quinolin-4-yl)cyclopentancarboxamid
N-[2-(Benzylamin)quinolin-4-yl]cyclopentancarboxamid
N-(2- [(4-Methylphenyl)amin] quinolin-4-yl)cyclopentqncqrboxamid
N-[2-(2,3-Dihydro-1H-inden-5-ylamin)quinolin-4-yl]cyclopentancarboxamid
N-(2- [(4-Methoxyphenyl)amin] quinolin-4-yl)cyclopentancarboxamid
N-(2- [(4-Chlorphenyl)amin] quinolin-4-yl)cyclopentancarboxamid
N- [2-(Cyclopentylamin)quinolin-4-yl]cyclopentancarboxamid
N-[2-(1H-indazol-6-ylamin)quinolin-4-yl]cyclopentancarboxamid
N-(2-Anilinquinolin-4-yl)cyclohexancarboxamid
N-(2- [(3,4-Dichlorphenyl)amin]quinolin-4-yl)cyclohexancarboxamid
N-(2- [(4-Methylphenyl)amin] quinolin-4-yl)cyclohexancarboxamid
N-[2-(2,3-Dihydro-1H-inden-5-ylamin)quinolin-4-yl]cyclohexancarboxamid
N-(2-Anilinquinolin-4-yl)benzamid
N-(2-[(3,4-Dichlorphenyl)amin]quinolin-4-yl]benzamid
N-(2-Anilinquinolin-4-yl)-2-furamid
N-(2-[(3,4-Dichlorphenyl)amin]quinolin-4-yl]-2-furamid
N-(2-Anilinquinolin-4-yl)cyclobutancarboxamid; und
N-(2-[(3,4-Dichlorphenyl)amin] quinolin-4-yl)cyclobutancarboxamid.

14. 2,4-disubstituiertes Quinolinderivat nach Anspruch 13, ausgewählt aus der Gruppe bestehend aus:
N-(2-[(3,4-Dichlorphenyl)amin] quinolin-4-yl)cyclopentancarboxamid
N-(2-[(4-Methoxyphenyl)amin] quinolin-4-yl)cyclopentancarboxamid
N-(2-[(4-Chlorphenyl)amin]quinolin-4-yl)cyclopentancarboxamid
N-(2-Anilinquinolin-4-yl)cyclohexancarboxamid
N-(2-[(3,4-Dichlorphenyl)amin] quinolin-4-yl)cyclocyclohexancarboxamid
N-(2-[(4-Methylphenyl)amin]quinolin-4-yl) cyclohexancarboxamid
N-[2-(2,3-Dihydro-1H-inden-5-ylamin)quinolin-4-yl] cyclohexancarboxamid; und
N-(2-[(3,4-Dichlorphenyl)amin] quinolin-4-yl)cyclobutancarboxamid.

15. 2,4-disubstituiertes Quinolinderivat nach Anspruch 14, ausgewählt aus der Gruppe bestehend aus:
N-(2-[(4-Chlorphenyl)amin]quinolin-4-yl)cyclopentancarboxamid
N-(2-Anilinquinolin-4-yl) cyclohexancarboxamid
N-(2-[(3,4-Dichlorphenyl)amin]quinolin-4-yl) cyclohexancarboxamid
N-[2-(2,3-Dihydro-1H-inden-5-ylamin)quinolin-4-yl] cyclohexancarboxamid

## Revendications

1. Modulateur allostérique du récepteur de l'adénosine (A₃RM), répondant à la formule générale (I) suivante : 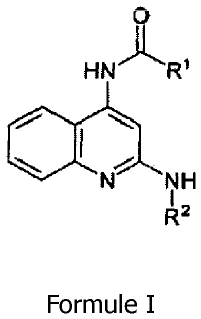 dans laquelle :
R₁ représente un groupe choisi parmi un groupe cycloalkyle en C₄ à C₁₂, un groupe cycloalcényle en C₄ à C₁₂, un groupe aryle en C₆ à C₁₂, un groupe hétéroaryle en C₄ à C₁₂, un groupe alkcycloalkyle, un groupe alkaryle, un groupe alkyle en C₁ à C₁₀, un groupe alcényle en C₂ à C₁₀ ; un groupe alcynyle en C₂ à C₁₀, un groupe cycloalkyle fusionné en C₅ à C₁₅, un noyau bicyclique aromatique ou hétéroaromatique ; un groupe alkyléther en C₁ à C₁₀, un groupe amino, un groupe hydrazido, un groupe alkylamino en C₁ à C₁₀, un groupe alcoxy en C₁ à C₁₀, un groupe alcoxycarbonyle en C₁ à C₁₀, un groupe alcanol en C₁ à C₁₀, un groupe acyle en C₁ à C₁₀, un groupe thioalcoxy en C₁ à C₁₀, un groupe pyridylthio, un groupe thio, et un groupe alkylthio en C₁ à C₁₀, un groupe acétoamido et un groupe acide sulfonique ;
R₂ représente un groupe choisi parmi un atome d'hydrogène ou un groupement cyclique choisi dans le groupe constitué des groupes aryle, hétéroaryle, alkaryle, alkhétéroaryle, cycloalkyle, hétérocycloalkyle, cycloalcényle, hétérocycloalcényle, cycloalcynyle, hétérocycloalcynyle, alkcycloalkyle, alkcyclohétéroalkyle,
alkcycloalcényle et alkhétérocycloalcényle, ledit groupement cyclique optionnellement substitué par au moins un groupe choisi parmi les groupes alkyle en C₁ à C₁₀, halogéno, alcanol en C₁ à C₁₀, hydroxyle, acyle en C₁ à C₁₀, alcoxy en C₁ à C₁₀ ; alcoxycarbonyle en C₁ à C₁₀, alcoxylalkyle en C₁ à C₁₀ ; thioalcoxy en C₁ à C₁₀ ; alkyléther en C₁ à C₁₀, amino, hydrazido, alkylamino en C₁ à C₁₀, pyridylthio, alcényle en C₂ à C₁₀ ; alcynyle en C₂ à C₁₀, thio, alkylthio en C₁ à C₁₀, acétoamido et acide sulfonique ; ou lesdits substituants peuvent former, conjointement avec un atome d'un groupement cyclique, un groupe cycloalkyle, hétérocycloalkyle, cycloalcényle ou hétérocycloalcényle fusionné au dit groupement cyclique ;
et des sels pharmaceutiquement acceptables de celui-ci,
pour l'utilisation dans le traitement d'une pathologie qui peut être traitée par un composé choisi dans le groupe constitué de l'adénosine, d'un agoniste du récepteur de l'adénosine A₃ ou d'un antagoniste du récepteur de l'adénosine A₃, dans lequel ladite pathologie est choisie parmi les maladies immunodépressives, un trouble hyperprolifératif, un trouble hyperplasique bénin, une maladie cutanée proliférative, une maladie inflammatoire, les affections ischémiques, et les affections associées à une pression intraoculaire élevée.

2. A₃RM pour une utilisation selon la revendication 1, dans lequel R¹ est choisi parmi les groupes cycloalkyle, aryle et hétéroaryle.

3. A₃RM pour une utilisation selon la revendication 1 ou 2, dans lequel R² est choisi parmi les groupes aryle, alkaryle, cycloalkyle, les groupes aryle ou cycloalkyle étant optionnellement substitués par au moins un substituant choisi parmi les groupes alkyle en C₁ à C₁₀, halogéno et alkyléther en C₁ à C₁₀.

4. A₃RM pour une utilisation selon l'une quelconque des revendications 1 à 3, dans lequel R¹ est choisi parmi
un groupe cycloalkyle en C₄ à C₆, un groupe phényle ou un noyau aromatique hétérocyclique à cinq chaînons répondant à la formule (II) suivante : 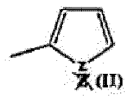 dans laquelle Z est choisi parmi un atome d'O, un atome de S ou un groupe NH ; et R² est choisi parmi un groupe cycloalkyle en C₄ à C₆, un groupe phényle, un groupe alkphényle, ou un noyau aromatique fusionné à un noyau cyclique ou hétéroaromatique à cinq chaînons répondant aux formules suivantes (IIIa) ou (IIIb) : 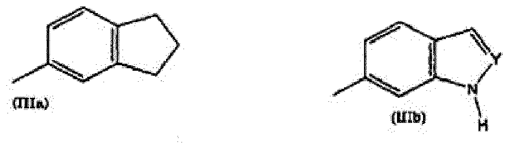 formules dans lesquelles Y est choisi parmi un atome de N ou un groupe CH ;
le noyau aryle ou cycloalkyle desdits groupes cycloalkyle, phényle et alkphényle ou desdites (Va) ou (Vb) étant optionnellement substitué par un substituant choisi parmi les groupes alkyle en C₁ à C₁₀, halogéno ou alkyléther en C₁ à C_{10.}

5. A₃RM pour une utilisation selon l'une quelconque des revendications 1 à 4, étant un dérivé de quinoline 2,4-disubstitué choisi parmi les composés de :
N-(2-anilinoquinolin-4-yl)cyclopentanecarboxamide
N-{2-[(3,4-dichlorophényl)amino]quinolin-4-yl}cyclopentanecarboxamide
N-[2-(benzylamino)quinolin-4-yl]cyclopentanecarboxamide
N-{2-[(4-méthylphényl)amino]quinolin-4-yl}cyclopentanecarboxamide
N-[2-(2,3-dihydro-1H-indén-5-ylamino)quinolin-4-yl]cydopentanecarboxamide
N-{2-[(4-méthoxyphényl)amino]quinolin-4-yl}cyclopentanecarboxamide
N-{2-[(4-chlorophényl)amino]quinoloin-4-yl}cyclopentanecarboxamide
N-[2-(cyclopentylamino)quinolin-4-yl]cyclopentanecarboxamide
N-[2-(1H-indazol-6-ylamino)quinolin-4-yl]cyclopentanecarboxamide
N-(2-anilinoquinolin-4-yl)cyclohexanecarboxamide
N-{2-[(3,4-dichlorophényl)amino]quinolin-4-yl}cyclohexanecarboxamide
N-{2-[(4-méthylphényl)amino]quinolin-4-yl}cyclohexanecarboxamide
N-[2-(2,3-dihydro-1H-indén-5-ylamino)quinolin-4-yl]cyclohexanecarboxamide
N-(2-anilinoquinolin-4-yl)benzamide
N-{2-[(3,4-dichloro-phényl)amino]quinolin-4-yl}benzamide
N-(2-anilinoquinolin-4-yl)-2-furamide
N-{2-[(3,4-dichlorophényl)amino]quinolin-4-yl}-2-furamide
N-(2-anilinoquinolin-4-yl)cyclobutanecarboxamide
N-{2-[(3,4-dichlorophényl)amino]quinolin-4-yl}cyclobutanecarboxamide.

6. A₃RM pour une utilisation selon l'une quelconque des revendications 1 à 5, dans lequel ladite utilisation est destinée à renforcer l'activité d'un récepteur de l'adénosine A₃ (A₃AR) pour ainsi traiter une pathologie qui peut être traitée à l'aide d'adénosine ou d'un agoniste de l'A₃AR, dans lequel ladite pathologie est choisie parmi un trouble hyperprolifératif, un trouble hyperplasique bénin, une maladie cutanée proliférative, une maladie inflammatoire, les affections ischémiques et les affections associées à une pression intraoculaire élevée.

7. A₃RM pour une utilisation selon la revendication 6 dans lequel ledit R¹ est un groupe cycloalkyle en C₄ à C₆ ou un groupe phényle ; et
R² est choisi parmi un groupe cycloalkyle en C₄ à C₆, un groupe phényle ou un noyau aromatique fusionné à un cycloalkyle à cinq chaînons répondant aux formules suivantes (III) : 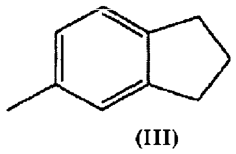 le groupement phényle de R² étant non substitué ou substitué au moins une fois par un groupe alkyle en C₁ à C₃, un atome d'halogène ou un groupe alkéther en C₁ à C₃.

8. A₃RM pour une utilisation selon la revendication 7, dans lequel ledit R¹ est choisi parmi les groupes cyclopentyle, cyclohexyle, cyclobutyle ou phényle; et
R² est choisi parmi un groupe cyclopentyle, un groupe phényle ou un noyau aromatique fusionné à un groupe cycloalkyle à cinq chaînons répondant aux formules suivantes (III) : 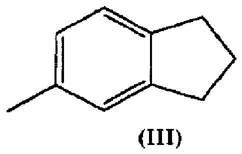 le groupement phényle de R² étant non substitué ou substitué au moins une fois par un groupe méthyle, un atome de Cl ou un groupe méthyléther.

9. A₃RM pour une utilisation selon la revendication 8, étant un dérivé de quinoline 2,4-disubstitué choisi parmi les composés de :
N-{2-[(3,4-dichlorophényl)amino]quinolin-4-yl}cyclopentanecarboxamide
N-{2-[(4-méthoxyphényl)amino]quinolin-4-yl}cyclopentanecarboxamide
N-{2-[(4-chlorophényl)amino]quinolin-4-yl}cyclopentanecarboxamide
N-(2-anilinoquinolin-4-yl)cyclohexanecarboxamide
N-{2-[(3,4-dichlorophényl)amino]quinolin-4-yl}cyclohexanecarboxamide
N-{2-[(4-méthylphényl)amino]quinolin-4-yl}cyclohexanecarboxamide
N-[2-(2,3-dihydro-1H-indén-5-ylamino)quinolin-4-yl]cyclohexanecarboxamide
N-{2-[(3,4-dichlorophényl)amino]quinolin-4-yl}cyclobutanecarboxamide.

10. A₃RM pour une utilisation selon la revendication 8, étant choisi parmi les composés de
N-{2-[(4-chlorophényl)amino]quinolin-4-yl}cyclopentanecarboxamide
N-(2-anilinoquinolin-4-yl)cyclohexanecarboxamide
N-{2-[(3,4-dichlorophényl)amino]quinolin-4-yl}cyclohexanecarboxamide
N-[2-(2,3-dihydro-1H-indén-5-ylamino)quinolin-4-yl]cyclohexanecarboxamide.

11. A₃RM pour une utilisation selon la revendication 10, étant du N-{2-[(3,4-dichlorophényl)amino]quinolin-4-yl}cyclohexanecarboxamide.

12. A₃RM pour une utilisation selon l'une quelconque des revendications 6 à 9, étant un renforçateur d'A₃AR destiné à renforcer l'activité de l'A₃AR, le renforcement comprenant l'occurrence d'un ou plusieurs des éléments suivants :
(a) une augmentation d'au moins 15 % de l'efficacité dudit A₃AR par liaison dudit renforçateur d'A₃AR à un site allostérique dudit A₃AR ; ou
(b) une diminution du taux de dissociation de l'adénosine ou d'un agoniste de l'A₃AR au niveau de son site de liaison.

13. Dérivé de quinoline 2,4-disubstitué choisi dans le groupe constitué de :
N-(2-anilinoquinolin-4-yl)cyclopentanecarboxamide
N-{2-[(3,4-dichlorophényl)amino]quinolin-4-yl}cyclopentanecarboxamide
N-[2-(benzylamino)quinolin-4-yl]cyclopentanecarboxamide
N-{2-[(4-méthylphényl)amino]quinolin-4-yl}cyclopentanecarboxamide
N-[2-(2,3-dihydro-1H-indén-5-ylamino)quinolin-4-yl]cyclopentanecarboxamide
N-{2-[(4-méthoxyphényl)amino]quinolin-4-yl}cyclopentanecarboxamide
N-{2-[(4-chlorophényl)amino]quinolin-4-yl}cyclopentanecarboxamide
N-[2-(cyclopentylamino)quinolin-4-yl]cyclopentanecarboxamide
N-[2-(1H-indazol-6-ylamino)quinolin-4-yl]cyclopentanecarboxamide
N-(2-anilinoquinolin-4-yl)cyclohexanecarboxamide
N-{2-[(3,4-dichlorophényl)amino]quinolin-4-yl}cyclohexanecarboxamide
N-{2-[(4-méthylphényl)amino]quinolin-4-yl}cyclohexanecarboxamide
N-[2-(2,3-dihydro-1H-indén-5-ylamino)quinolin-4-yl]cyclohexanecarboxamide
N-(2-anilinoquinolin-4-yl)benzamide
N-{2-[(3,4-dichloro-phényl)amino]quinolin-4-yl}benzamide
N-(2-anilinoquinolin-4-yl)-2-furamide
N-{2-[(3,4-dichlorophényl)amino]quinolin-4-yl}-2-furamide
N-(2-anilinoquinolin-4-yl)cyclobutanecarboxamide ; et
N-{2-[(3,4-dichlorophényl)amino]quinolin-4-yl}cyclobutanecarboxamide.

14. Dérivé de quinoline 2,4-disubstitué selon la revendication 13, choisi dans le groupe constitué de :
N-{2-[(3,4-dichlorophényl)amino]quinolin-4-yl}cyclopentanecarboxamide
N-{2-[(4-méthoxyphényl)amino]quinolin-4-yl}cyclopentanecarboxamide
N-{2-[(4-chlorophényl)amino]quinolin-4-yl}cyclopentanecarboxamide
N-(2-anilinoquinolin-4-yl)cyclohexanecarboxamide
N-{2-[(3,4-dichlorophényl)amino]quinolin-4-yl}cyclohexanecarboxamide
N-{2-[(4-méthylphényl)amino]quinolin-4-yl}cyclohexanecarboxamide
N-[2-(2,3-dihydro-1H-indén-5-ylamino)quinolin-4-yl]cyclohexanecarboxamide,et
N-{2-[(3,4-dichlorophényl)amino]quinolin-4-yl}cyclobutanecarboxamide.

15. Dérivé de quinoline 2,4-disubstitué selon la revendication 14, choisi dans le groupe constitué de :
N-{2-[(4-chlorophényl)amino]quinolin-4-yl}cyclopentanecarboxamide
N-(2-anilinoquinolin-4-yl)cyclohexanecarboxamide
N-{2-[(3,4-dichlorophényl)amino]quinolin-4-yl}cyclohexanecarboxamide
N-[2-(2,3-dihydro-1H-indén-5-ylamino)quinolin-4-yl]cyclohexanecarboxamide.
